# EUROPEAN PATENT SPECIFICATION

(11) **EP 4 009 893 B1**
(45) Date of publication and mention of the grant of the patent: **01.10.2025**
(21) Application number: 20761406.6
(22) Date of filing: 07.08.2020
(51) Int. Cl.: A61B 18/02

(54) **SYSTEMS AND DEVICES FOR ABLATION AND DEFUNCTIONALIZATION OF A GALLBLADDER**
SYSTEME UND VORRICHTUNGEN ZUR ABLATION UND DEFUNKTIONALISIERUNG EINER GALLENBLASE
SYSTÈMES ET DISPOSITIFS D'ABLATION ET DE PERTE DE FONCTIONNALITÉ D'UNE GALLE

(30) Priority: 07.08.2019 US 201962884001 P
(43) Date of publication of application: 15.06.2022
(73) Proprietor: Ictero Medical, Inc., Houston, Texas 77021 (US)
(72) Inventor: NOJOOMI, Matthew, Houston, Texas 77007 (US); WATERS, David, Houston, Texas 77007 (US); BESSOFF, Kovi, Palo Alto, California 94304 (US); RYBA, Eric, Durango, Colorado 81301 (US)
(74) Representative: Cooley (UK) LLP
(86) International application number: PCT/US2020/045436
(87) International publication number: WO 2021/026467

(56) References cited:
- WO-A2-2018/106688
- US-A1- 2009 036 823
- US-A1- 2010 331 883
- US-A1- 2016 038 212

## Description

### CROSS-REFERENCE

This application claims the benefit of U.S. Provisional Application No. 62/884,001, filed August 7, 2019, titled "Cryogen Supply Valve and Pressure Relief Control for Cryoablation Devices".

### BACKGROUND

Medical ablation technologies, such as those used in cardiology, oncology, general surgery, gastroenterology, dermatology, and interventional radiology, focus on local tissue targets and, while providing a great degree of ablation depth control, may not be effective or practical for large, high-surface area (HSA) tissue ablation targets within a body. Cryoablation technologies leverage a generic cryogen spray to provide a platform for HSA tissue ablation, but have certain drawbacks associated with safely and effectively delivering energy within closed lumens, such as the gallbladder. For example, ice build-up or other complications during an ablation procedure can lead to injury and/or ineffective ablation. Accordingly, it is desirable to have systems, devices, and methods to address the drawbacks of existing ablation systems.
US2009/036823 discloses a device and method for safely securing a multilumen device to a tissue, organ or solid tumor within the body of a human during a diagnostic or therapeutic procedure. The device is capable of securing the tumor by touching or piercing its surface and providing a coolant to the distal tip. Cooling the tip forms a cryogenically induced region that tightly binds the tip to the tumor, temporarily sealing the entry-track of the instrument. The device further provides at least one injecting/aspirating lumen that can dispense or aspirate a fluid within the tumor. Such construction allows injecting part or the whole volume of the tumor while the cryogenically induced bond prevents back-flow of the injected substances through the entry-track.

### SUMMARY OF THE INVENTION

The invention is defined by the appended independent claim. Further embodiments of the invention are illustrated in the dependent claims. The methods illustrated herein do not form part of the invention, but are useful for understanding the invention.

### SUMMARY OF THE DISCLOSURE

The present disclosure relates to devices and methods for ablation and defunctionalization of a gallbladder. In some embodiments, an apparatus includes a shaft including a nozzle disposable within a body lumen of a subject, the shaft defining: an ablation medium delivery lumen in fluid communication with the nozzle, the ablation medium delivery lumen configured to deliver an ablation medium to the nozzle, the nozzle configured to distribute the ablation medium throughout the body lumen such that the ablation medium contacts and ablates tissue within the body lumen; a first pressure lumen having a distal opening that terminates in the body lumen when the nozzle is disposed in the body lumen, the first pressure lumen configured to couple a first pressure sensor disposed proximal to the shaft to the body lumen such that the first pressure sensor can measure an intraluminal pressure of the body lumen; and a second pressure lumen having a distal opening that terminates in an evacuation lumen configured to evacuate a portion of the ablation medium from the body lumen, the second pressure lumen configured to couple a second pressure sensor disposed proximal to the shaft to the evacuation lumen such that the second pressure sensor can measure a pressure in the evacuation lumen.

In some embodiments, an apparatus includes an outer shaft defining an evacuation lumen, the outer shaft having a distal portion disposable within a body lumen of a subject, the evacuation lumen configured to evacuate an ablation medium from the body lumen; an inner shaft including a nozzle configured to distribute the ablation medium throughout the body lumen such that the ablation medium contacts and ablates tissue within the body lumen, the inner shaft disposable within the evacuation lumen and configured to extend out from the evacuation lumen such that the nozzle is disposed distal to a distal end of the outer shaft, the inner shaft defining: an ablation medium delivery lumen in fluid communication with the nozzle, the ablation medium delivery lumen configured to deliver the ablation medium to the nozzle such that the nozzle can distribute the ablation medium throughout the body lumen; and a sensor lumen having a distal opening that terminates in the body lumen when the nozzle is disposed distal to the distal end of the outer shaft, the sensor lumen configured to couple a sensor to the body lumen such that the sensor can measure a property associated with the body lumen.

In some embodiments, an apparatus includes a shaft having a distal end that is disposable within a body lumen of a subject, the shaft defining (1) an ablation medium delivery lumen configured to deliver an ablation medium into the body lumen and (2) a pressure lumen having a distal opening that terminates in the body lumen when the distal end of the shaft is disposed within the body lumen; a pressure sensor coupled to the body lumen via the pressure lumen, the pressure sensor configured to measure an intraluminal pressure of the body lumen; a valve disposed within the ablation medium delivery lumen, the valve configured to transition between an open state to allow the ablation medium to the be delivered into the body lumen and a closed state to prevent the ablation medium from being delivered into the body lumen; and a control unit operatively coupled to the pressure sensor and the valve, the control unit configured to control the valve based on the intraluminal pressure measured by the pressure sensor.

In some embodiments, a method includes receiving an ablation fluid into a delivery lumen of a first shaft having a distal end disposed in a gallbladder lumen of a subject, conveying, via the delivery lumen of the first shaft, the ablation fluid to the distal end of the first shaft; dispensing the ablation fluid from the distal end of the first shaft such that the ablation fluid transitions into an ablation gas that contacts and ablates tissue within the gallbladder lumen; evacuating, via an evacuation lumen of a second shaft, at least a portion of the ablation gas from the gallbladder lumen; measuring an intraluminal pressure of the gallbladder lumen via a pressure sensor operatively coupled to a pressure lumen of the first shaft, the pressure lumen having a distal opening that terminates in the gallbladder lumen when the distal end of the first shaft is disposed in the gallbladder lumen; and controlling delivery of the ablation fluid to the distal end of the first shaft based on the intraluminal pressure of the gallbladder lumen.

### BRIEF DESCRIPTION OF THE DRAWINGS

FIG. 1 is a schematic illustration of an ablation system, according to an embodiment.
FIG. 2 is a schematic illustration of an outer shaft of a catheter system, according to an embodiment.
FIG. 3 is a schematic illustration of an inner shaft of a catheter system, according to an embodiment.
FIG. 4 is a schematic illustration of a catheter system, according to an embodiment.
FIG. 5 is a schematic illustration of a control unit of an ablation system, according to an embodiment.
FIG. 6 is an illustration of an ablation system implemented as a cryoablation device, including control for monitoring and/or controlling intraluminal pressure during cryogen delivery, according to an embodiment.
FIG. 7 is flow chart of a method for ablation and intraluminal pressure control, according to an embodiment.
FIG. 8 is a flow chart of a method for temperature control when using an ablation system (e.g., cryoablation device), according to an embodiment.
FIG. 9 is a flow chart of a method for pressure control when using an ablation system (e.g., cryoablation device), according to an embodiment.
FIGS. 10A-10C are illustrations of different percutaneous and endoscopic access approaches for ablation systems, according to an embodiment.
FIG. 11A-11D are illustrations of an ablation system (e.g., cryoablation device) with a pressure sensor, according to various embodiments.
FIG. 12A-12D are illustrations of an ablation system (e.g., cryoablation device) with multiple pressure sensors, according to various embodiments.
FIG. 13 is a schematic illustration of pressure control for an ablation system (e.g., cryoablation device), according to an embodiment.
FIG. 14 is an illustration of an inner shaft with a fenestrated nozzle, according to an embodiment.
FIG. 15 is an illustration of an ablation system including a temperature measurement probe, according to an embodiment.
FIG. 16 is an illustration of an ablation system including a temperature measurement probe, according to an embodiment.
FIG. 17 is an illustration of an inner shaft with an ablation medium supply valve, according to an embodiment.
FIG. 18 is an illustration of an inner shaft with an ablation medium supply valve, according to an embodiment.
FIG. 19 is an illustration of an inner shaft with an ablation medium supply valve, according to an embodiment.
FIG. 20 shows pressure operation parameters under different conditions, according to an embodiment.
FIGS. 21A-21B shows operating pressure data for a cryoablation device based on lumen size and flow rate for an ablation system (e.g., a cryoablation device).

### DETAILED DESCRIPTION

The present disclosure relates to ablation systems, devices, and methods for ablating a body lumen, such as, for example, a gallbladder lumen. In some embodiments, systems, devices, and methods described herein relate to cryoablation devices for tissue ablation. In some embodiments, systems, devices, and methods described herein relate to ablation medium release valves, e.g., for catheter-based cryoablation devices, that are designed to safely, effectively, and uniformly disperse an ablation medium (e.g., a cryogenic ablation medium) onto an area of interest (e.g., tissue lining a gallbladder lumen). In some embodiments, systems, devices, and methods described herein relate to controlling an operation of an ablation system based on sensor data, e.g., pressure and/or temperature data. In some embodiments, systems, devices, and methods described herein include sensors and/or can be used with sensors (e.g., of one or more probes) to track properties or conditions of a body lumen (e.g., a gallbladder lumen) and/or an ablation medium being delivered into the body lumen. Examples of suitable components of ablation systems, including cryoablation devices, are described in International Patent Application No. PCT/US2019/017112, entitled "GALLBLADDER DEFUNCITONALIZATION DEVICES AND METHODS," filed on February 7, 2019.

Gallstones are one of the most common gastrointestinal disorders amongst Americans. Gallstones form when bile, a fluid secreted by the liver and stored in the gallbladder, becomes supersaturated. While they do not cause a problem for many people, gallstones occasionally block the cystic duct, i.e., an outlet of the gallbladder, preventing the gallbladder from emptying. In some instances, the obstruction results in pain, inflammation, and infection. In otherwise healthy patients, the gallstone disease is treated by surgical removal of the gallbladder. However, the risks associated with surgical treatment are considerably higher in certain patient populations. For example, one in five Medicare patients have been shown to suffer an adverse outcome. Non-surgical treatment options for these patients are limited and focus on relieving acute symptoms, without addressing the underlying cause of the disease. In some instances, the disease is likely to recur, resulting in additional clinical risk and significant cost. There currently is no long-term solution for gallbladder disease in high-risk patients.

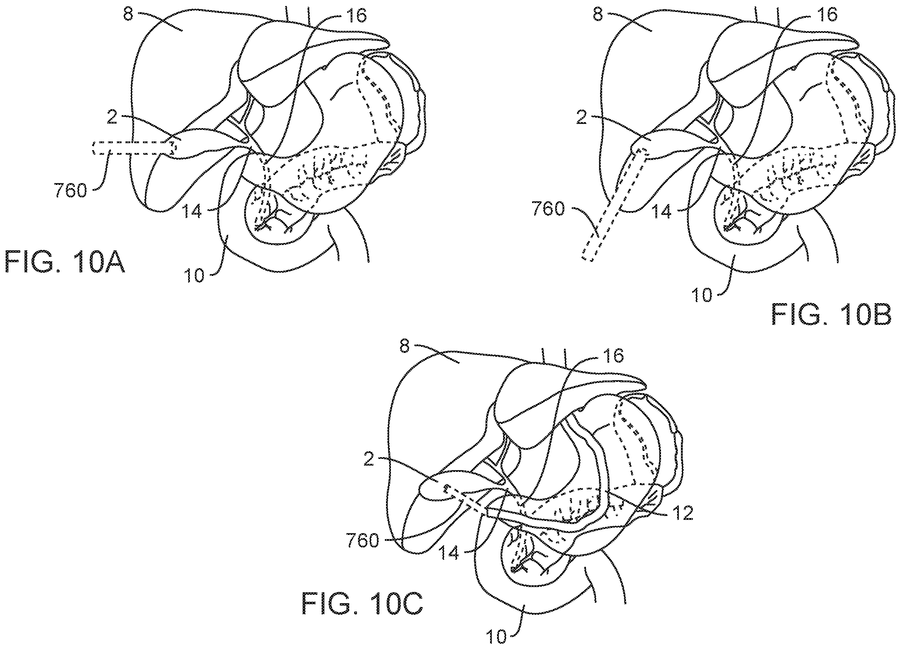

As depicted in FIGS. 10A-10C, the gallbladder 2 is a small hollow organ in the gastrointestinal system. A blind-ended tubular outpouching of the biliary tree, the gallbladder 2 is a pear-shaped organ with a storage capacity of 30 milliliters (mL) - 50 mL. The gallbladder is typically 2-3 centimeters (cm) in breadth and 7-10 cm in axial length. The gallbladder is typically divided into three parts: the fundus, body, and neck. The neck contains a mucosal fold, known as Hartmann's Pouch, which is a common location for gallstones to become lodged, resulting in cholecystitis. As shown in FIGS. 10A-10C, the gallbladder 2 opens into a cystic duct 14 and connects to the liver 8 by the common hepatic duct 18 which bifurcates into the right hepatic duct and the left hepatic duct. The gallbladder 2 is connected to the small intestine 10 by the common bile duct 16.

Histologically, the gallbladder has 4 layers, including the serosa (the outermost layer), a muscular layer, lamina propria, and the innermost mucosa layer. The mucosal layer of the gallbladder is the innermost layer of the gallbladder wall and concentrates the bile. The serosa is derived from the visceral peritoneum and covers the anterior fundus, body, and neck of the gallbladder. Inside the serosa, a single muscular layer envelopes the lamina propria. The mucosa that lines the inner lumen of the gallbladder is composed of columnar epithelial cells which secrete mucin and dehydrate bile via the action of multiple ion channels. Occasionally, outpouchings (known as Rokitansky-Aschoff nodules) of the mucosa extend into deeper layers of the gallbladder wall.

The gallbladder stores and concentrates the bile produced by the liver and releases the stored bile into the small intestine, where the bile helps in the digestion of fats in food. Bile is made by hepatocytes in the liver and subsequently secreted into hepatic ductules which coalesce into intrahepatic ducts. These ducts converge to form the right and left hepatic ducts which then combine into the common bile duct. The common bile duct joins with the pancreatic duct just proximal to the ampulla of Vater in the duodenal wall. Bile produced by hepatocytes flows through the biliary system and into the duodenal lumen to aid in digestion.

Flow into the duodenal lumen is regulated at the level of the ampulla of Vater by the sphincter of Oddi. During an unfed state, when bile is not needed for digestion, the sphincter is closed, resulting in routing of bile to the gallbladder for storage. During storage, bile becomes supersaturated, providing a nidus for the formation of gallstones and sludge (very small gallstones). The majority of gallstones are "brown stones," that are mainly comprised of cholesterol (typically >80%). These stones tend to be brittle and are readily crushed. A minority of stones are predominantly bilirubin ("black stones"; <20% cholesterol) and are often much harder. Mixed stones contain a variable amount of bilirubin and cholesterol.

Mobile gallstones that remain in the lumen of the gallbladder have the potential to cause various pathologies. In some instances, the gallstones become lodged at the neck of the gallbladder, occluding the cystic duct. The lodged gallstones cause gallbladder distension and intermittent right upper quadrant discomfort (likely from intramural muscle spasm at the organ attempts to empty against an increased pressure gradient), a condition known as symptomatic cholelithiasis. In some instances, the gallstones become lodged more permanently at the gallbladder outlet, resulting in inflammation and infection. This is a condition known as cholecystitis, which requires urgent intervention as it can progress to systemic infection.

Alternatively or in combination, gallstones or sludge passes through the cystic duct, becoming lodged in the common bile duct, blocking the flow of bile, resulting in a potentially life threatening condition known as ascending cholangitis. In some embodiments, the debris becomes lodged at the confluence of the pancreatic and common bile ducts, causing stagnation of pancreatic secretions, resulting in pancreatitis (inflammation of the pancreas).

In cholelithiasis, supersaturation of bile in gallbladder leads to the formation of gallstones. In some instances, impacted gallstones leads to inflammation, pain and infection of the gallbladder. When the gallbladder is inflamed, the mucosal layer of the gallbladder becomes more prominent. In some instances, the gallstone disease is diagnosed by ultrasounds or other imaging methods. Provided herein are methods and devices configured to definitively treat benign gallbladder disease in a minimally invasive manner in patients with symptomatic gallstones in order to reduce health care costs and patient morbidity.

Laparoscopic cholecystectomy is a treatment for gallstone disease and is a commonly performed general surgery procedure. During laparoscopic cholecystectomy, small incisions are made in the abdomen, facilitating the removal of the gallbladder with a camera and small instruments. The procedure is safe in otherwise healthy patients, and often does not require hospital admission. In uncomplicated cases, patients are often back to work within two weeks.

In a number of patient populations, the surgical risk associated with laparoscopic cholecystectomy is considerably higher. In some instances, these populations include critically ill patients, patients with intra-abdominal scarring from chronic disease and previous surgery, and elderly patients who tend to have a higher incidence of medical comorbidities. One such population is the Medicare population, which comprises approximately 200,000 laparoscopic cholecystectomies per year in the US. Twenty one percent of these surgeries result in an adverse outcome, including prolonged length of stay and readmission and other perioperative complications. In addition to the direct costs associated with these complications, many elderly patients are at risk of not returning to their baseline level of health, resulting in additional healthcare costs.

There are non-surgical options to treat gallstone disease. These include the administration of antibiotics, or placement of a cholecystostomy tube to drain the gallbladder contents, or a combination of the two. However, the non-surgical options do not provide a long-term solution. These options are effective temporizing measures, and they do not treat the cause of the disease. During a percutaneous cholecystostomy, a cholecystostomy tube is placed through the rib cage into the gallbladder. The percutaneous cholecystostomy can take place in an interventional radiology (IR) suite or at the patient's bedside but does not provide a definite treatment of the gallstone disease. Often times, the non-surgical options lead to recurrence and additional hospitalization costs.

For patients with cholecystitis who have a high risk of surgical complications, the treatment is percutaneous decompression of the gallbladder (via a percutaneously inserted cholecystostomy tube) in conjunction with antibiotics. This treatment provides a temporizing measure to allow the patient to recover from the systemic effects of the ongoing infection (sepsis) and return to their baseline state of health (commonly referred to as "cooling off' by healthcare professionals). The cholecystostomy tube remains in place until the patient has recovered. About 6-8 weeks following placement, a cholangiography by injection of radiopaque contrast through the tube under fluoroscopy is performed to determine if the cystic duct is patent (open). The cholecystostomy tube is removed if the cystic duct is patent. The treatment is interval cholecystectomy as it reduces the rate of recurrence of the gallstone disease. If there is no communication between the cystic duct and the common bile duct, the tube remains in place until cholecystectomy is performed, or patency is demonstrated on subsequent cholangiography. There is no definitive treatment available for high risk patients, placing them at risk for disease recurrence and exposure to the associated clinical risks and healthcare costs.

Ablation technologies have been used to treat other diseases. For example, ablation has been used in treatment of esophageal metaplasia and endometrial hyperplasia. However, ablation technologies are not readily available for treating gallstone disease. Ablation technologies often are applied to a small targeted area, such as a nerve, and are not typically used for applying to a diffuse area or a tissue or organ. Systems, devices, and methods described herein relate to ablating and defunctionalizing a gallbladder, and are specifically designed to safely and efficiently ablate the gallbladder.

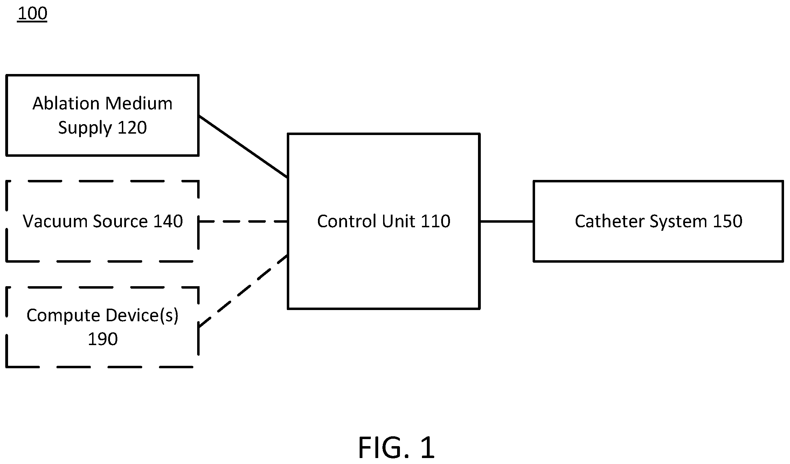

FIG. 1 is a schematic illustration of an example ablation system 100, according to an embodiment. The ablation system 100 includes a control unit 110, a catheter system 150, and an ablation medium supply 120. The control unit 110 can control the operation of one or more components of the ablation system 100.

The control unit 110 can be operatively coupled to the ablation medium supply 120, which provides a supply of an ablation medium. For example, the control unit 110 can be configured to control delivery of the ablation medium into a body lumen (e.g., gallbladder lumen). In some embodiments, the ablation medium is a cryogenic ablation medium. In some embodiments, the cryogenic ablation medium is a liquid. In some embodiments, the cryogenic ablation medium is a gas. In some embodiments, the cryogenic ablation medium undergoes a liquid-to-gas phase transition when being delivered using the systems and devices disclosed herein. In some embodiments, cryoablation is achieved via the refrigerant property due to the liquid to gas phase change from an ablation medium, such as liquid nitrous oxide, carbon dioxide, and argon. In some embodiments, the cryogenic ablation medium is one or more of nitrous oxide, nitrogen, carbon dioxide, or argon. In some embodiments, the cryogenic ablation medium can transition from a first state (e.g., a liquid) to a second state (e.g., a gas) and increase up to about 600 times an original volume of the cryogenic medium during the transition. In some embodiments, the control unit 110 can control one or more of a temperature, a pressure, etc. of the ablation medium. In some embodiments, an ablation medium such as a cryogenic ablation medium ranges from about -120 degrees Celsius to about 0 degrees Celsius, including all values and subranges in between, when the cryogenic ablation medium is used with the systems and devices disclosed herein.

The control unit 110 can optionally be coupled to a vacuum source 140 (e.g., a vacuum or suction pump, an aspirator, etc.). In some embodiments, the control unit 110 can control the vacuum source 140 to apply a vacuum to a channel or lumen of the catheter system 150, e.g., to remove or evacuate an ablation medium from within a body lumen (e.g., gallbladder lumen). For example, the control unit 110 can activate the vacuum source 140 to apply negative pressure within a lumen of the catheter system 150 to evacuate a portion of an ablation medium, such as a cryogenic ablation medium, that has been delivered to the body lumen. Alternatively, in some embodiments, the ablation system 100 does not include a vacuum source 140, and ablation medium can be evacuated from a body lumen via passive evacuation driven by a pressure differential between an interior of the body lumen and an exterior environment. For example, when an ablation medium such as a cryogenic ablation medium is delivered into a body lumen, the ablation medium can increase pressure within the body lumen relative to an environment exterior to the body lumen (e.g., an exterior atmosphere), and that pressure differential can drive evacuation of a portion of the ablation medium out of the body lumen, e.g., via a lumen defined by the catheter system 150.

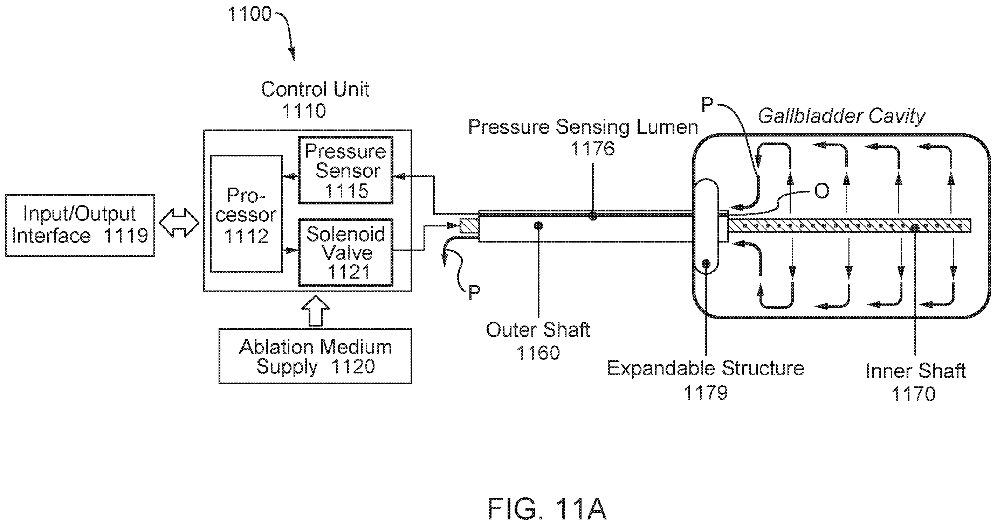

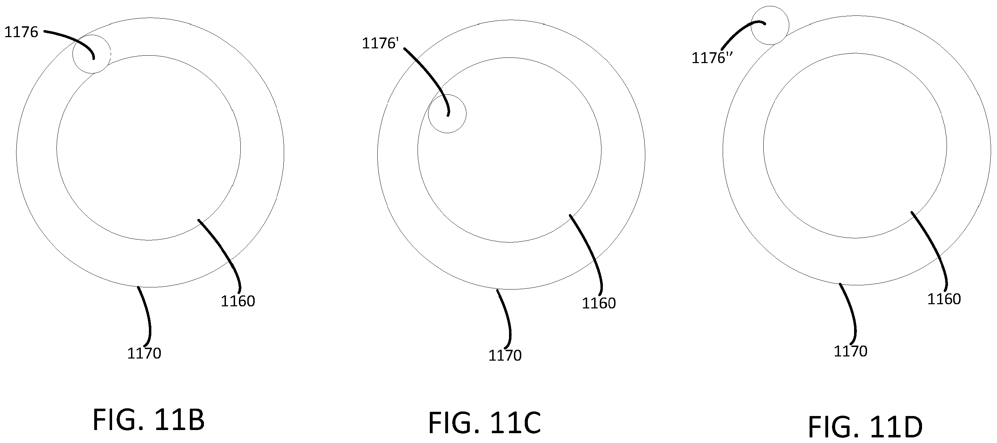

In some embodiments, the control unit 110 can include or be operatively coupled to one or more sensors (e.g., pressure sensors, temperature sensors), and can operate or control one or more components of the ablation system 100 based on data collected by the one or more sensors. For example, the control unit 110 can be coupled to a pressure sensor and, based on measurements from the pressure sensor, control delivery of the ablation medium (e.g., from ablation medium supply 120) and evacuation of the ablation medium (e.g., using vacuum source 140) to maintain pressure within a body lumen within a predetermined range of pressures. Stated differently, the control unit 110 can be configured to control insufflation of a body lumen such that pressure within the lumen is maintained within a predetermined range of pressures. In some embodiments, the predetermined pressure range is less than 50 mmHg, or less than 100 mmHg. In some embodiments, the predetermined pressure range is about 0 mmHg to about 40 mmHg, or about 30 mm Hg to about 40 mm Hg. In some embodiments, the control unit 110 can be operatively coupled to one or more valves, which the control system 110 can control to allow and/or terminate delivery or evacuation of an ablation medium. Further details of such control mechanisms are described with reference to FIGS. 11-13 and 17-19.

In some embodiments, the control unit 110 and/or other components of the ablation system 100 can optionally be coupled to one or more additional compute devices 190. Compute device(s) 190 can be can be any suitable processing device configured to run and/or execute certain functions. The one or more compute device(s) 190 can include, for example, a computer, a laptop, a portable device, a mobile device, or other suitable compute device including a processor, a memory, and/or an input/output device. For example, the control unit 110 can be coupled to a remote compute device, such as a workstation, through which a user (e.g., physician, administrator, etc.) can control one or more operational parameters of the ablation system 100. The control unit 110 and the one or more compute device(s) 190 can be configured to send data and/or receive data from one or more other compute device(s) 190, e.g., via a network. For example, the control unit 110 can send alerts and/or other information to a remote device (e.g., a display, a mobile device) such that the remote device can present that information to a user (e.g., a physician). In some embodiments, the control unit 110 can send data such as patient information, operational status of one or more components of the ablation system 100, etc.

In some embodiments, the control unit 110 can be integrated into a handheld device that is attached to a proximal end of the catheter system 150. The handheld device can include on or more input and/or output devices (e.g. buttons, switches, keyboards, touchscreens, display, etc.) through which an operator of the ablation system 100 can control the operation of the ablation system 100 to perform an ablation procedure. In some embodiments, the control unit 110 can be remote from the catheter system 150, and a remote operator can control one or more components of the ablation system 100 to perform an ablation procedure.

The catheter system 150 can be percutaneously inserted into a body lumen (e.g., a gallbladder lumen) to scar down and defunctionalize portions of anatomy (e.g., the gallbladder) without the need for surgical removal of the anatomy. The catheter system 150 can be used in the interventional radiology (IR) suite and with local anesthesia, eliminating the risks associated with general anesthesia in high risk surgical patients. Placement of the device leverages existing IR workflows and can be deployed in a manner similar to existing devices. For example, for placement in the gallbladder lumen, such placement can be similar to a cholecystostomy tube or percutaneous gallbladder drainage tube.

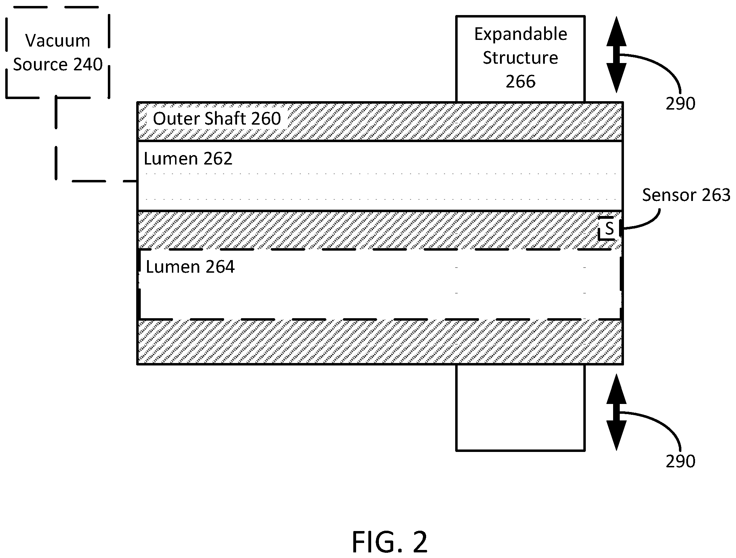

FIGS. 2-4 provide schematic views of portions of an example catheter system 250, according to some embodiments. Catheter system 250 can be structurally and/or functionally similar to catheter system 150. For example, catheter system 250 can be coupled to a control unit (e.g., control unit 110) and/or be configured to receive an ablation medium from an ablation medium supply (e.g., ablation medium supply 220). The catheter system 250 can include an outer shaft 260 and an inner shaft 270. The inner shaft 270 can also be referred to as a first shaft, and the outer shaft 260 can be referred to as a second shaft. In some embodiments, the outer shaft 260 and the inner shaft 270 can be separate components that are used together, e.g., to perform an ablation procedure. For example, the outer shaft 260 can be implemented as an access sheath or introducer, and the inner shaft 270 can be implemented as a catheter that is insertable into a lumen of the introducer. In some embodiments, the outer shaft 260 and the inner shaft 270 can be integrated into a single catheter device, e.g., a device having two concentric shafts.

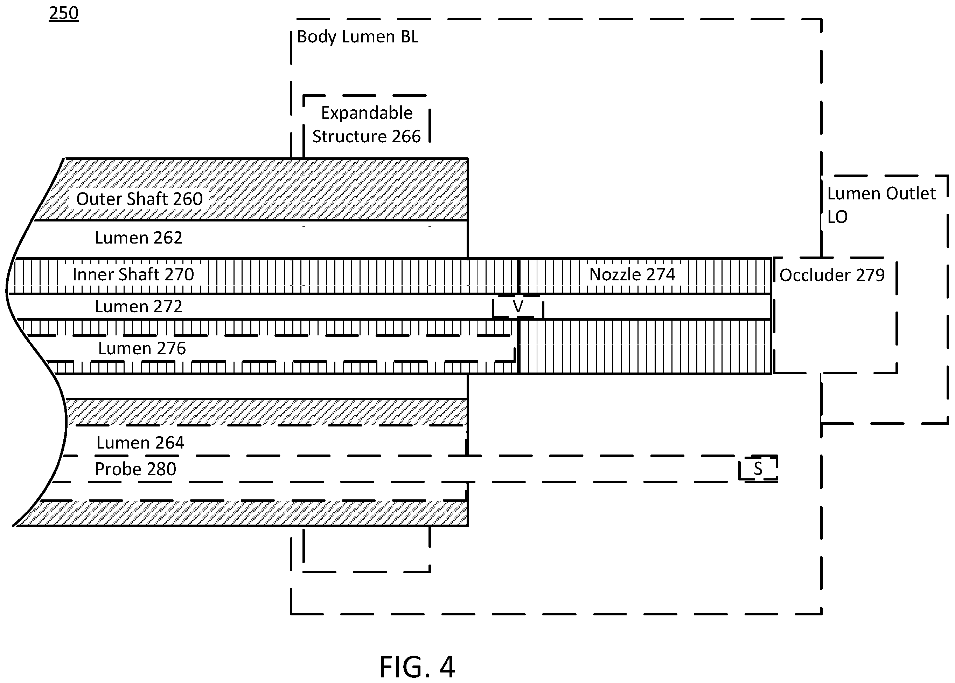

FIG. 2 provides a detailed view of the outer shaft 260. The outer shaft 260 can be, for example, an access sheath. The outer shaft 260 can define a lumen 262. The lumen 262 can be configured to receive one or more instruments, including, for example, the inner shaft 270. The outer shaft 260 can be configured to provide access to a body lumen BL (e.g., a gallbladder lumen). For example, a distal end of the outer shaft 260 can be positioned within a body lumen BL, as schematically depicted in FIG. 4. Once positioned inside the body lumen BL, the outer shaft 260 can enable delivery of one or more instruments into the body lumen BL, e.g., via lumen 262. For example, as depicted in FIG. 4, the inner shaft 270 can be inserted into lumen 262 of the outer shaft 260 and navigated into the body lumen BL such that a distal end of the inner shaft 270 is positioned inside of the body lumen BL.

In some embodiments, the lumen 262 can be configured to evacuate or drain fluids (e.g., liquids or gases) and/or debris (e.g., gallstones or fragments thereof, tissue, etc.) from within the body lumen BL. For example, the lumen 262 can allow an ablation medium (e.g., a cryogenic ablation medium) delivered to the body lumen BL to be evacuated from the body lumen BL. In some embodiments, the lumen 262 can be operatively coupled to a vacuum source 240, which can be activated to apply negative pressure within lumen 262 to evacuate fluid from within the body lumen BL. Alternatively, the lumen 262 can function as a passive evacuation passageway for fluid to exit the body lumen BL. For example, as ablation medium is delivered into the body lumen BL and pressure increases within the body lumen BL relative to an exterior of the shaft 260, such pressure can passively drive a portion of the ablation medium out from the body lumen BL via lumen 262.

In some embodiments, the outer shaft 260 can define one or more additional lumens, e.g., a lumen 264, which can be structurally and/or functionally similar to lumen 262. For example, lumen 264 can also be configured to provide access into the body lumen BL. In some embodiments, lumen 262 can be configured to receive the inner shaft 270 and lumen 264 can be configured to receive a different surgical and/or monitoring device (e.g., a probe, a second ablation device, etc.). In some embodiments, one or more of lumens 262, 264 can be fluidically coupled to a sensor (e.g., a pressure sensor) to allow for pressure measurements of the body lumen BL and/or other portions of the body. For example, a sensor integrated into a control unit (e.g., control unit 110) can be in fluid communication with one or more of lumens 262, 264 and take measurements (e.g., pressure measurements) of an environment within the outer shaft 260 and/or body lumen BL.

In some embodiments, the outer shaft 260 optionally includes a sensor 263. In some embodiments, the sensor 263 can be located in a distal portion of the outer shaft 260 that is configured to be disposed within the body lumen BL. Alternatively, the sensor 263 can be disposed at a different location along the outer shaft 260, including, for example, within a lumen (e.g., lumen 262, 264), at a proximal end at the outer shaft 260, etc. The sensor 263 can be configured to capture information about an environment within the body lumen BL or other environment within and/or surrounding the outer shaft 260. For example, the sensor can be configured to measure a property (e.g., pressure, temperature) of an ablation medium being delivered to the body lumen BL, a property (e.g., pressure, temperature) of the body lumen BL or fluid within the body lumen BL, etc. The sensor 263 can include, for example, a pressure sensor (e.g., pressure transducer, strain gauge transducer, diaphragm displacement sensor, optical fiber pressure sensor, solid state sensor), temperature sensor, light sensors, gas sensors, etc. In some embodiments, sensor 263 can be coupled to a control unit (e.g., control unit 110) and/or other compute device (e.g., compute device 190) via a wired connection, such as, for example, a wire that is coupled to and/or disposed within the outer shaft 260. In some embodiments, the sensor 263 can be configured to wirelessly transmit data, e.g., indicative of one or more measured properties of the body lumen BL, to control unit and/or another compute device.

In some embodiments, the outer shaft 260 includes an expandable member or structure 266 that can be deployed within the body lumen BL, e.g., transitioned from an undeployed sate to a deployed or expanded state. The expandable structure 266 can be configured to prevent dislodgement and/or create a seal between the outer shaft 260 and the body lumen BL. In use, the outer shaft 260 can be advanced, e.g., along a guidewire, until a distal end of the outer shaft 260 is positioned within the body lumen BL through an opening. The expandable structure 266 can then be deployed (e.g., expanded, inflated), as schematically shown in FIG. 2 by arrows 290. Once deployed (e.g., once in its deployed state), the expandable structure can have a diameter larger than an diameter of the opening through which the outer shaft 260 has been placed and therefore be configured to retain the outer shaft 260 within the body lumen BL. In some embodiments, the expandable structure 266 includes an inflatable balloon, a shape memory structure (e.g., a deployable nitinol structure), etc. In some embodiments, the expandable structure 266 in its deployed state can have an outer diameter that is about 1.5 times to about 3 times larger than an outer diameter of the outer shaft 260. Further details of suitable expandable structures 266, such as, for example, a seal, are described in International Patent Application No. PCT/US2019/017112.

While two lumens (e.g., lumens 262, 264) are depicted in FIG. 2, it can be appreciated that the outer shaft 260 can include any number of lumens, including a single lumen and/or more than two lumens. The outer shaft 260 can also include additional sensors, expandable structures, etc. according to embodiments described herein.

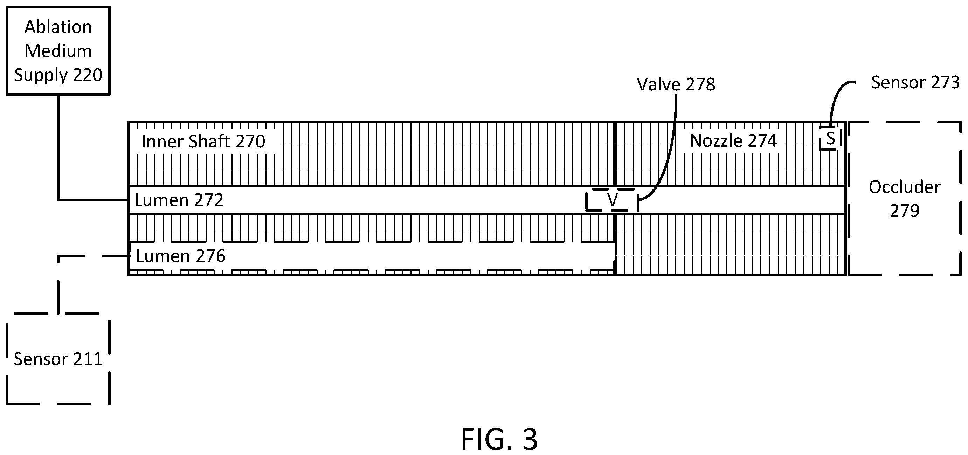

FIG. 3 provides a more detailed view of the inner shaft 270. The inner shaft 270 can be, for example, an ablation delivery device or ablation catheter. In some embodiments, the inner shaft 270 can form a portion of a cryoablation device and be configured to deliver a cryogenic ablation medium into the body lumen BL. The inner shaft 270 can be configured to provide an ablative energy or an ablative medium capable of killing cells within the body lumen BL. For example, the inner shaft 270 can be configured to provide an ablative energy or ablative medium capable of killing cells in a mucosal layer of a gallbladder lumen, killing cells lining a cystic duct, or any combination thereof. The ablative energy or medium can include, for example, a chemical agent (e.g., an antibiotic, a liquid sclerosant, sodium tetradecyl sulphate, acetic acid, ethanol, hypertonic sodium chloride, urea), a cryogenic ablation medium (e.g., a cryogenic liquid or gas), thermal ablation, electrical ablation, etc. In some embodiments, the inner shaft 270 can be configured to deliver multiple types of ablative energies or mediums. The inner shaft 270 can be configured to provide ablation that is spatially diffuse. Stated differently, the inner shaft 270 can be configured to provide ablation that ablates a large area of a body lumen BL. In some embodiments, the inner shaft 270 can be configured to deliver ablation for defunctionalizing gallbladder mucosa, for ablating or sclerosis of a cystic duct, or any combination thereof.

In some embodiments, the inner shaft 270 can be configured to deliver thermal ablation, cryoablation, chemical ablation, or any combination thereof. In some embodiments, cryoablation involves delivering a low temperature fluid to wall of the gallbladder, such as liquid nitrogen. In some embodiments, cryoablation involves delivering an ablation medium to the gallbladder wall that induces low temperatures due to phase change, such as nitrous oxide or carbon dioxide. In some embodiments, thermal ablation involves delivering a high temperature fluid to the wall of the gallbladder, such as, for example, hot water or steam. In some embodiments, the ablative medium is delivered in a liquid form, a gaseous form, an aerosol form, a gel form, or any combination thereof.

The inner shaft 270 can define a lumen 272. The lumen 272 can be configured to deliver an ablation medium, e.g., from ablation medium supply 220, to a nozzle 274 that is disposable within the body lumen BL. The nozzle 274 can be configured to release the ablation medium into the body lumen BL. In some embodiments, the nozzle 274 can include a plurality of openings or fenestrations for distributing the ablation medium throughout the body lumen BL. In some embodiments, the lumen 272 and nozzle 274 can be configured to convey a cryogenic ablation medium in a liquid state into the body lumen BL. The lumen 272 and nozzle 274 can be configured with dimensions that maintain a set amount of pressure on the cryogenic ablation medium such that the medium does not undergo a liquid-to-gas transition until the ablation medium exits the openings of the nozzle 274. Stated differently, the lumen 272 and nozzle 274 can be configured to convey a cryogenic ablation medium in a liquid state to the openings of the nozzle 274, at which point the release of the cryogenic ablation medium into the body lumen BL results in the cryogenic ablation medium changing from the liquid state into a gas state. In some embodiments, the lumen 272 of the inner shaft 270 can have a diameter from about 0.0254 mm to about 2.54 mm (about 0.001 inches to about 0.1 inches), including all values and subranges in between.

In some embodiments, the inner shaft 270 can optionally include a valve 278. The valve 278 can be configured to control delivery of the ablation medium into the body lumen BL. For example, the valve 278 can be configured to turn on or shut off supply of the ablation medium into the nozzle 274. In some embodiments, a control unit (e.g., control unit 110) can be configured to control opening and/or closing of the valve 278. In some embodiments, a mechanical actuator (e.g., coupled to a handheld device, as described above) can be used to open and/or close the valve 278. In some embodiments, the valve 278 can be configured to close (e.g., automatically and/or via control by a control unit) in response to a pressure within the body lumen BL being greater than a predetermined threshold. In some embodiments, a sensor (e.g., sensor disposed on inner or outer shaft 260, 270 and/or sensor coupled to control unit 110) can be used to measure the pressure within the body lumen BL and control the valve 278 to open and/or close. In some embodiments, the valve 278 can be configured to close in response to a pressure difference between the body lumen BL and an evacuation lumen (e.g., lumen 262), e.g., indicating that a blockage or obstruction has formed along an evacuation pathway. For example, multiple sensors can be configured to measure different pressures associated with the catheter system 250 and/or body lumen BL, and a control unit (e.g., control unit 110) can be configured to analyze when such pressure measurements to determine when an unexpected obstruction has isolated any fluid flow paths into and/or out of the body lumen BL.

As noted above, in some embodiments, the inner shaft 270 can be or form part of a cryoablation device and be configured to deliver a cryogenic ablation medium into the body lumen BL. The cryoablation device can leverage the phase-change properties of certain cryogenic ablation mediums (e.g., liquid nitrous oxide) to induce cryoablation temperatures at a target tissue interface. When such cryogenic ablation mediums transition from liquid to gas, they expand in volume and can cause increase in pressure within the body lumen BL. Therefore, one important consideration in designing systems and devices disclosed herein lies in the monitoring and control of the intraluminal pressure in the body lumen BL during an ablation procedure. For example, systems and devices disclosed herein can be configured to ensure that intraluminal pressure does not increase above a predetermined threshold and/or lies within a predetermined range. In instances where there is an increase in intraluminal pressure (e.g., pressure above a predetermined threshold, or sudden change in pressure above a predetermined rate), systems and devices disclosed herein can be configured to evacuate air, gaseous cryoablation medium, and/or other fluids from within the body lumen BL to reduce the intraluminal pressure. In such instances, it can be important to ensure to any cryogenic ablation medium within the catheter system 250 (e.g., within lumen 272 of the inner shaft 270) and/or supply line into the catheter system 250 does not exit the catheter system 250 (e.g., nozzle 274) into the body lumen BL, further adding to the pressure increase. Accordingly, it can be desirable to minimize or reduce the amount of residual cryogenic ablation medium that is delivered into the body lumen BL in response to detecting a pressure increase event (e.g., pressure above a predetermined threshold, or sudden change in pressure above a predetermined rate). In some embodiments, the valve 278 can be used to reduce the amount of residual cryogenic ablation medium that is delivered into the body lumen 270. The valve 278 can be positioned at or near the nozzle 274 such that the valve 278, upon closing, prevents any residual or excess ablation medium within the lumen 272 and/or other passageways leading to the nozzle 274 from being delivered into the body lumen BL.

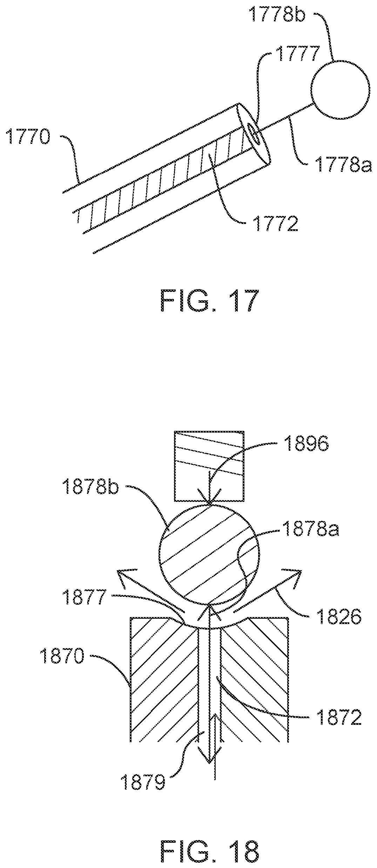

The valve 278 can include any range of suitable mechanisms. In some embodiments, the valve can be closed in its resting state but can open to allow ablation medium to be delivered into the body lumen BL. Alternatively, the valve 278 can be open in its resting state and can be closed to prevent additional ablation medium from being delivered into the body lumen BL. In some embodiments, the valve 278 can be biased closed and/or open using a spring mechanism. The valve 278 can have any suitable geometry including, for example, a cube, cone, cylinder, triangular prism, torus, helix, ovoid, or other three-dimensional body with sufficient structure to impede ablation medium flow. In some embodiments, the valve 278 can be seated against a valve seat defined within the inner shaft 270 (e.g., within lumen 272). In some embodiments, the valve 278 can be actuated, either manually or via a control device (e.g., control device 110), with a drive wire or rod, pneumatic or hydraulic pressure, electromagnetic force, and/or motor to open and/or close. Further details of example valve designs are described with reference to FIGS. 17-19.

In some embodiments, the inner shaft 270 optionally includes a sensor 273. In some embodiments, the sensor 273 can be located in a distal portion of the inner shaft 270 that is configured to be disposed within the body lumen BL. Alternatively, the sensor 273 can be disposed at a different location along the inner shaft 270, including, for example, within a lumen (e.g., lumen 272, 276), at a proximal end at the inner shaft 270, etc. The sensor 273 can be configured to capture information about an environment within the body lumen BL. For example, the sensor 273 can be configured to measure a property (e.g., pressure, temperature) of an ablation medium being delivered to the body lumen BL, a property (e.g., pressure, temperature) of the body lumen BL or fluid within the body lumen BL, etc. The sensor 273 can include, for example, a pressure sensor (e.g., pressure transducer, strain gauge transducer, diaphragm displacement sensor, optical fiber pressure sensor, solid state sensor), temperature sensor, light sensors, gas sensors, etc. Sensor 273 can be capable of communicating data (e.g., sensor measurements) to a control unit (e.g., control unit 110) and/or other compute device (e.g., compute device 190) via a wired or wireless connection.

In some embodiments, the inner shaft 270 can optionally include one or more additional lumens 276. In some embodiments, a lumen 276 can configured as a passageway for relaying pressure information or other conditions (e.g., temperature) from the body lumen BL and/or other portions of the catheter system 250. For example, a first lumen 276 can be configured to extend or open into the body lumen BL, and a pressure sensor 211 (e.g., a sensor disposed or operatively coupled to a control unit, such as control unit 110) can be used to measure a pressure within the body lumen BL (i.e., the intraluminal pressure). As another example, a temperature sensor can be used to measure temperature within the body lumen via the lumen 276. A second lumen (not depicted) can be configured to extend or open into the evacuation lumen (e.g., lumen 262), and a pressure sensor can be used to measure a pressure within the evacuation lumen. In some embodiments, the lumen 276 can be configured to receive a guidewire and/or one or more additional instruments (e.g., temperature, pressure, or other sensor probes).

In some embodiments, the catheter system 250 can optionally include an occluder 279. Occluder 279 can be configure to occlude or close an opening or lumen outlet LO into nearby anatomical structures from the body lumen BL. For example, in the case where the body lumen BL is a gallbladder lumen, the occluder 279 can be configured to occlude a cystic duct. The occluder 279 can be coupled to and/or detachable from the inner shaft 270. In operation, the occluder 279 can be coupled to a distal end of the inner shaft 270. The inner shaft 270 can be navigated into the body lumen BL, e.g., via insertion through the outer shaft 260. The inner shaft 270 can be manipulated to position the occluder 279 at an opening out of the body lumen BL (e.g. outlet lumen such as a cystic duct). The occluder 279 can then be decoupled or ejected from the inner shaft 270, allowing the occluder 279 to be placed in the opening. The occluder 279 can subsequently be fixed in place, e.g., via volume expansion of the occluder 279, external threads, friction fit, adhesion, or other suitable fixation mechanism. Further details of suitable occluders such as, for example, plugs, are described in International Patent Application No. PCT/US2019/017112.

While two lumens (e.g., lumens 272, 276) are depicted in FIG. 3, it can be appreciated that the inner shaft 270 include any number of lumens, including a single lumen and/or more than two lumens. The inner shaft 270 can also include additional sensors, valves, nozzles, etc. according to embodiments described herein.

FIG. 4 provides a detailed view of the inner shaft 270 and the outer shaft 260 positioned within the body lumen BL. The inner shaft 270 can be disposed within the lumen 262 of the outer shaft 260. The spacing between an outer surface of the inner shaft 270 and an inner surface of the lumen 262 can define an evacuation lumen or passageway for removing gas and/or other fluids from the body lumen BL (e.g., ablation medium from the body lumen BL). The occluder 279, with the inner shaft 270 so positioned, can be disposed in a lumen outlet LO (e.g., a cystic duct). Also shown in FIG. 4 is a probe 280, which can be optionally positioned in the lumen 264 of the outer shaft 260. The probe 280 can be a sensor probe that is used to measure a property of the body lumen and/or an ablation medium that is delivered into the body lumen BL.

The outer shaft 260, inner shaft 270, and/or probe 280 can be formed of flexible and/or semi-flexible material that enables each to be navigated to the body lumen BL, e.g., along a guidewire. The material can be a medical grade, biocompatible material.

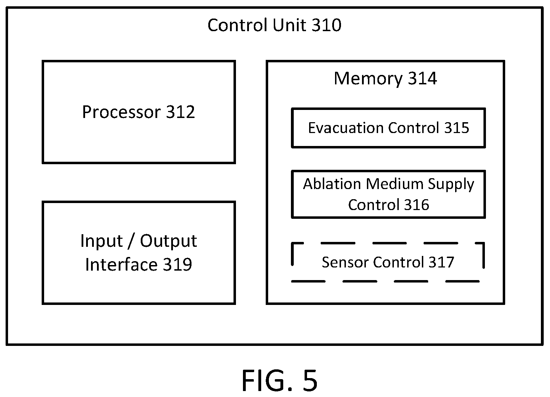

FIG. 5 is a schematic illustration of an example control unit 310, according to some embodiments. Control unit 310 can be structurally and/or functionally similar to control unit 110, as described with reference to FIG. 1. For example, control unit 310 can be configured to control one or more components of an ablation system and/or catheter system (e.g., ablation system 100, catheter system 250). Control unit 310 can include a processor 312, a memory 314, and an input/output interface 319. In some embodiments, the control unit 310 can be coupled to the catheter system, e.g., by being contained in a handheld device that is coupled to a proximal end of the catheter system. In some embodiments, the control unit 310 can be remotely situated, e.g., on a remote compute device or system, and can be used to remotely control the operation of the catheter system.

Processor 312 of control unit 310 can be any suitable processing device configured to run and/or execute functions associated with deploying one or more components of a catheter system (e.g., advancing or retracting a shaft, deploying an expandable structure, opening and/or closing a valve), delivering ablation medium into a body lumen, analyzing sensor data associated with an ablation procedure involving the catheter system, controlling temperature and/or pressure within the body lumen, etc. Processor 312 can be configured to execute modules, functions, and/or processes. Processor 312 can be a general purpose processor, a Field Programmable Gate Array (FPGA), an Application Specific Integrated Circuit (ASIC), a Digital Signal Processor (DSP), and/or the like. In some embodiments, processor 312 is part of a circuit, e.g., such as an integrated circuit. In some embodiments, one or more other components of the ablation system can be integrated into the circuit, including, for example, one or more sensors.

Input/output interface 319 can include a user interface and/or communication interfaces for connecting the control unit 310 to one or more external compute devices. The user interface(s) can include one or more components that are configured to receive inputs and send outputs to other devices and/or a user operating a device, e.g., a user operating a catheter system. For example, the user interface can include a display device (e.g., a display, a touch screen, etc.), an audio device (e.g., a speaker or alarm), and one or more additional input/output device(s) configured for receiving an input and/or generating an output to a user. The communication interface(s) can include one or more wireless and/or wired interfaces, e.g., for communicating with other compute device (e.g., compute device(s) 190) via one or more networks (e.g., a local area network (LAN), a wide area network (WAN), a virtual network, a telecommunications network).

Memory 314 can be, for example, a random access memory (RAM), a memory buffer, a hard drive, a database, an erasable programmable read-only memory (EPROM), an electrically erasable read-only memory (EEPROM), a read-only memory (ROM), and/or so forth. In some embodiments, memory 314 stores instructions that cause processor 312 to execute modules, processes, and/or functions associated with deploying one or more components of a catheter system (e.g., advancing or retracting a shaft, deploying an expandable structure, opening and/or closing a valve), delivering ablation medium into a body lumen, analyzing sensor data associated with an ablation procedure involving the catheter system, controlling temperature and/or pressure within the body lumen, etc. Methods as described herein can be implemented by way of machine (e.g., computer processor) executable code stored on an electronic storage location of the control unit 310, such as, for example, on the memory 314, or a memory operatively coupled to the control unit 310. In some embodiments, the machine executable or machine-readable code is provided in the form of software. In operation, the code can be executed by the processor 312. In some cases, the code is retrieved from the memory 314 to be accessed and/or executed by the processor 312.

As depicted in FIG. 5, memory 314 stores instructions that can cause processor 312 to execute modules, processes, and/or functions, illustrated as evacuation control 315, ablation medium supply control 316, and/or optionally sensor control 317. Evacuation control 315, ablation medium supply control 316, and/or sensor control 317 can be implemented as one or more programs and/or applications that are tied to hardware components. For example, evacuation control 315, ablation medium supply control 316, and/or sensor control 317 can be implemented by one or more components of an ablation system and/or catheter system (e.g., ablation system 100, catheter system 250). In some embodiments, the processor 312 executing evacuation control 315 can control the opening of a valve and/or activation of a vacuum source to evacuate gas or other fluid (e.g., ablation medium) from a body lumen. In some embodiments, the processor 312 executing ablation medium supply control 316 can control the opening of a valve and/or operation of an ablation supply source to deliver an ablation medium into a body lumen via a catheter system. In some embodiments, the processor 312 executing sensor control 317 can receive, process, and/or analyze data from one or more sensors and/or use such data to control the operation of one or more other components of the ablation system or catheter system.

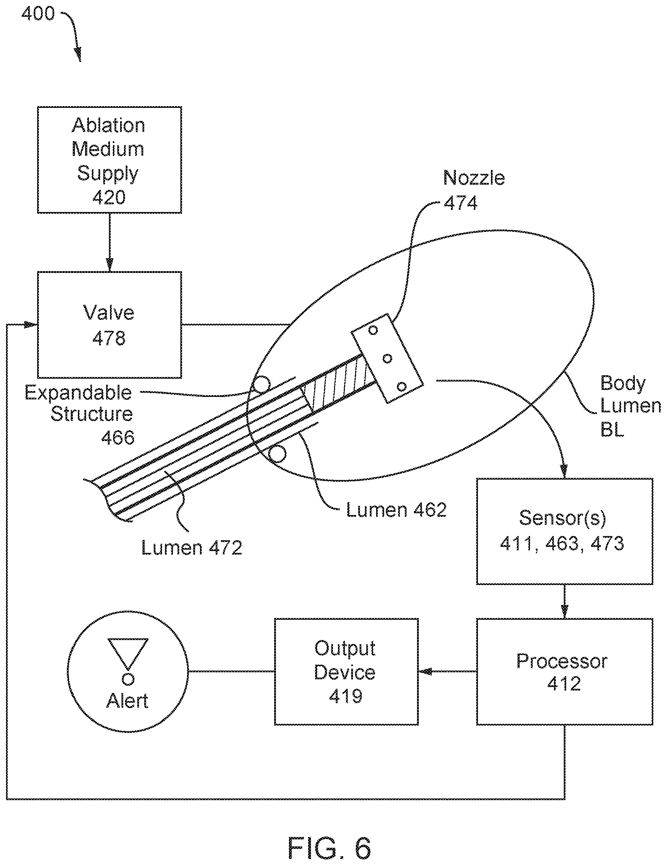

FIG. 6 depicts an example ablation system 400, including control circuitry and logic for monitoring and controlling intraluminal pressure during an ablation procedure. The ablation system 400 can be structurally and/or functionally similar to other ablation systems described herein, such as, ablation system 100, and can include a catheter system (e.g., structurally and/or functionally similar to catheter system 250) and a control unit (e.g., structurally and/or functionally similar to control unit 310). In some embodiments, one or more sensors (e.g., sensors 411, 463, 473, structurally and/or functionally similar to sensors 211, 263, 273) intermittently or continuously measure one or more properties of a body lumen BL (e.g., a pressure or temperature) and relay measurements to a processor 412 (e.g., structurally and/or functionally similar to processor 312) which implements a feedback loop as depicted in FIG. 6. More specifically, FIG. 6 shows a an ablation system 400 with a feedback loop for controlling intraluminal pressure buildup during an ablation procedure. In an embodiment, the ablation system 400 can be a cryoablation device that delivers a cryogenic ablation medium into a gallbladder lumen. The ablation system 400 can include an ablation supply medium 420, a valve 478, outer and inner shafts that define lumens 462, 472, respectively, an expandable structure 466, and a nozzle 474. In some embodiments, one or more sensors 411, 463, 473 intermittently or continuously measure one or more properties associated with the body lumen BL (e.g., pressure, temperature) and relay measurements to a processor 412.

In the event a pressure buildup or increase is detected (e.g., pressure being above a threshold value or changing a predetermined percentage or amount), a user interface can alert the user, e.g., via an output device 419, with an audio, visual and/or haptic response. Alternatively, in some embodiments, the user is not notified but the processor 412 controls a valve 478 to terminate delivery of the ablation medium into the body lumen. In some embodiments, in the event of a pressure buildup event, the processor 412 can detect the event and directly interface with (e.g., control) the valve 478 to close within a predetermined period of time (e.g., about 0-10 seconds response time, inclusively), such that the flow of ablation medium into the body lumen BL is terminated. In at least one embodiment, one or more additional valves (not depicted) disposed closer to or at the ablation medium supply 420 can be used to shut off the flow of ablation medium from the ablation medium supply 420 into the lumen 472 (e.g., of a catheter system) as a secondary control measure.

In at least one embodiment, the space in the lumen 462 between the outer shaft and the inner shaft provides an evacuation path for gas volume within the target lumen. In at least one embodiment, one or more active and/or passive evacuation mechanisms, as described above with respect to FIGS. 1-5, can be applied to remove gas and/or other fluid, including, for example, the ablation medium, from the body lumen BL.

In at least one embodiment, the inner and/or outer shaft can be held stationary during an ablation procedure. In another embodiment, the at least a portion of the inner and/or outer shafts can be dynamic and move around within a two-dimensional plane and/or three-dimensional volume within the body lumen.

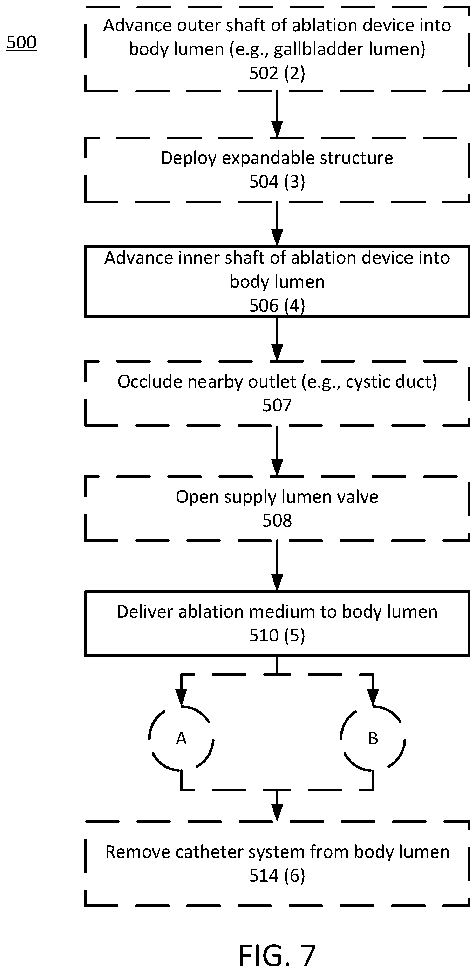

FIGS. 7-9 depict an example method 500 for ablation and managing pressure and/or temperature during an ablation procedure, according to an embodiment. In some embodiments, the ablation procedure can be a cryoablation procedure that is implemented using an ablation system (e.g., ablation system 100) that includes a cryoablation device. In some embodiments, the ablation procedure is performed in the gallbladder to defunctionalize the gallbladder. The method 500 optionally includes advancing an outer shaft (e.g., outer shaft 260) of a catheter system (e.g., catheter system 150, 250) of the ablation system into a body lumen (e.g., gallbladder lumen), at 502. In some embodiments, ultrasonic imaging can be used to visualize the body lumen. In some embodiments, a standard needle and guidewire can be used to access the body lumen via a trans-hepatic approach, e.g., using the Seldinger technique. When the target body lumen is the gallbladder, visual return of bile through the needle and fluoroscopic confirmation of the guidewire curling inside the body lumen can assist with validating proper placement of a guidewire within the body lumen. In some embodiments, a series of progressively larger dilators can be advanced along the guidewire to dilate the tract into the body lumen. After dilating the tract, the outer shaft can be advanced along the guidewire into the body lumen. In some embodiments, the outer shaft of the catheter system can be a separate tubular structure, e.g., an access sheath or an introducer, that is first placed within the body lumen before placing an inner shaft within the body lumen. Alternatively, in some embodiments, the outer and inner shafts can be placed into the body lumen simultaneously. In some embodiments, placement of the outer shaft can be similar to a percutaneous drainage tube placement technique.

In some embodiments, the catheter system can be placed into a gallbladder lumen. Accessing the gallbladder with the catheter system can be achieved through a percutaneous approach. In some embodiments, an access sheath or outer shaft 760 of the catheter system accesses the gallbladder 2 through a transhepatic, percutaneous approach using ultrasound guidance, as seen in FIG. 10A. In some embodiments, the access sheath 760 of the catheter device accesses the gallbladder 2 through a subhepatic, percutaneous approach using ultrasound guidance, as seen in FIG. 10B. In some embodiments, the percutaneous approach is similar to the method used to place a cholecystostomy drain. In some embodiments, the access sheath 760 provided herein accesses the gallbladder 2 endoscopically, as shown in FIG. 10C. In some embodiments, the access sheath 760 accesses the gallbladder 2 utilizing native anatomy by creating a transmural stoma connecting the inner lumen of the gallbladder to the lumen of the small bowel, as shown in FIG. 10C. In some embodiments, percutaneous access is gained using a hollow bore needle, whereby a guidewire is placed through the needle to create a tract to the desired access location (e.g., a cystic duct, a gallbladder, or a combination thereof). In some embodiments, the access sheath 760 and an inner shaft or ablation catheter are configured with a concentric lumen to enable a guidewire to pass through. In some embodiments, the access sheath 760 and the ablation catheter are configured with a non-concentric lumen to enable a guidewire to pass through.

After positioning the distal end of the outer shaft of the catheter system within the body lumen, the method 500 can optionally include deploying an expandable structure, at 504. Deploying the expandable structure within the body lumen can ensure that the outer shaft (e.g., access catheter, introducer) remains or is retained within the body lumen during the ablation procedure.

The method 500 can include advancing an inner shaft (e.g., inner shaft 270) of the catheter system into the body lumen, at 506. The inner shaft can be advanced until a nozzle (e.g., nozzle 274) of the inner shaft is disposed within the body lumen distal to a distal end of the outer shaft. The inner shaft can be advanced into the body lumen by inserting the inner shaft into a lumen defined by the outer shaft and advancing the inner shaft through that lumen until a distal portion of the inner shaft is disposed distal to the outer shaft. The distal portion of the inner shaft can include one or more openings (e.g., fenestrations) that can deliver ablation medium into the body lumen. In some embodiments, the method 500 can optionally include deployment of saline to lavage and drain any content within the body lumen, e.g., via inner and/or outer shafts. For example, fluid such as saline can be delivered into the gallbladder via a first lumen (e.g., lumen 272 defined by inner shaft 270) and/or content within the body lumen (e.g., gallbladder content) can be evacuated from the body lumen via a second lumen (e.g., lumen 262 defined by outer shaft 260).

The method 500 can optionally include occluding a nearby outlet of the body lumen (e.g., a cystic duct), at 507. For example, an occluder (e.g., occluder 279) can be positioned at the outlet lumen and deployed (e.g., ejected, expanded, etc.) to occlude the outlet lumen. The method 500 can optionally include opening a supply lumen valve (e.g., valve 278), at 508. For example, as discussed above with reference to FIG. 3, a valve can be positioned along an ablation medium delivery passageway (e.g., along lumen 272 defined by inner shaft) to control delivery of the ablation medium. The valve in its open state can allow ablation medium to flow past the valve and into the body lumen, while the valve in its closed state can block the flow of ablation medium into the body lumen. In some embodiments, the valve can naturally be in a closed state, and therefore method 500 can include opening the valve such that ablation medium can be delivered into the body lumen. In some embodiments, the valve can naturally be in an open state, and therefore 508 can be omitted.

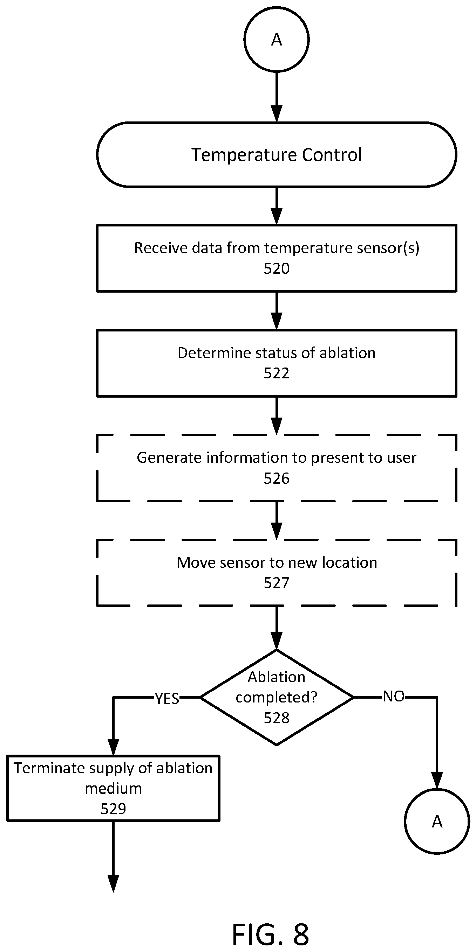

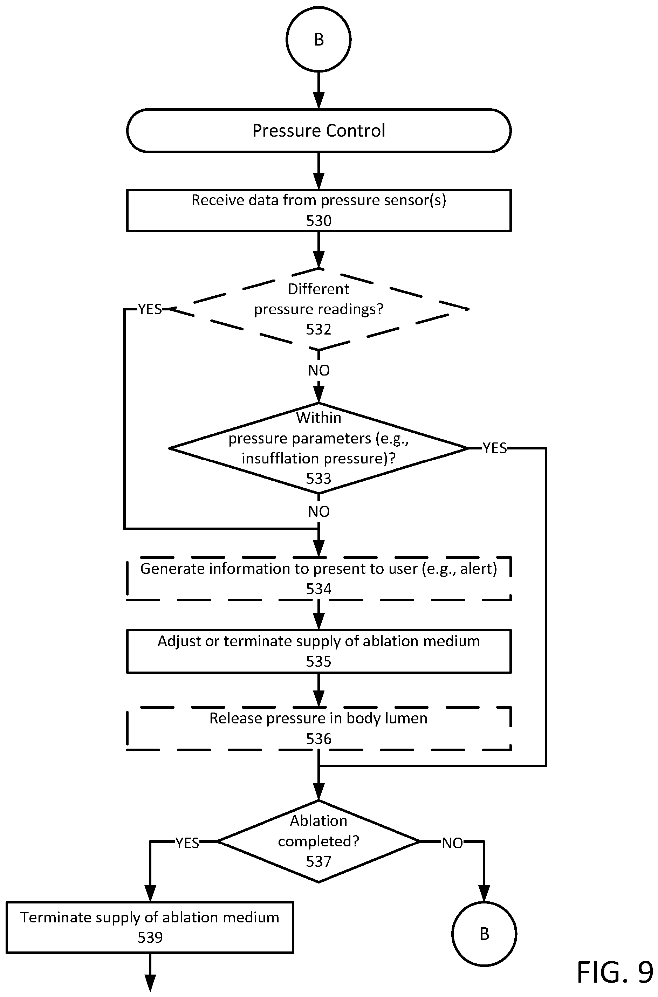

The method 500 can include delivering the ablation medium to the body lumen, at 510. In some embodiments, a cartridge (e.g., ablation medium supply source 120, 220) of a cryogenic ablation medium (e.g., nitrous oxide) or any other suitable ablation medium can be loaded into a handle (e.g., handheld device) of the ablation device. In the case of using a cryogenic ablation medium, 1, 2, 3, 4, 5, 6, 7, 8, 9, 10 or more than 10 freeze-thaw cycles can be delivered to the gallbladder, at 510, to ensure complete hypothermic death of the gallbladder mucosa. While delivering the ablation medium, method 500 can include events and/or steps associated with pressure and/or temperature monitoring, as further described below with reference to FIGS. 8 and 9. For example, a control unit (e.g., control unit 110, 310) can monitor pressure, temperature, and/or other conditions to ensure safe delivery of the ablation medium. After delivering the ablation medium, the method 500 can optionally include removing the catheter system (e.g., inner and outer shafts) from the body lumen, at 514.

FIG. 8 illustrates events and steps associated with a temperature monitoring protocol performed during an ablation procedure, such as the ablation procedure described with reference to FIG. 7. The control unit (e.g., control unit 110, 310) of the ablation system or another compute device can be configured to receive data from a temperature sensor, at 520, and determine a status of ablation, at 522. In some embodiments, the status of the ablation can be determined by data received from the temperature sensor. In some embodiments, the temperature sensor can be integrated into one of the inner or outer shafts (e.g., as sensor 263, 273). In other embodiments, the temperature sensor can be operatively coupled to a lumen (e.g., lumen 276) that extends into the body lumen, which the sensor can use to measure a temperature associated with the boy lumen. In still other embodiments, the temperature sensor can be mounted to a probe (e.g., probe 280) that is separately insertable into the body lumen, e.g., via a separate lumen (e.g., lumen 264) and/or the same lumen that houses the inner shaft (e.g., lumen 262). In some embodiments, the temperature sensor can be implanted or inserted into tissue within the body lumen such that a temperature of the tissue can be measured.

The method 500 can optionally include generating information to present to a user, at 526. In some embodiments, the information presented to the user can be presented via a compute device, such as control unit 110 and/or 310, or other compute device in network communication with the ablation system (e.g., a tablet, smartphone, or any other suitable communication device). In some embodiments, to evaluate the temperature of tissue at a different location, the temperature sensor can optionally be moved to a new location, at 527. For example, the temperature sensor can be retracted from tissue a first location, moved to a second location, and inserted into tissue at a second location. At step 528, the method 500 can include determining if the ablation is completed based at least on the temperature measurements collected by the temperature sensor. If the ablation is completed (528: YES), the supply of ablation medium is terminated at 529, and then the method 500 can continue to 514. If the ablation is not completed (528: NO), the method can continue to monitor the temperature and status of ablation, by repeating 520-527.

FIG. 9 illustrates events and steps associated with a pressure control protocol performed during an ablation procedure, such as the ablation procedure described with reference to FIG. 7. The control unit (e.g., control unit 110, 310) or another compute device can receive pressure data from one or more pressure sensors, at 530. In some embodiments, a first pressure reading can be from inside the body lumen (e.g., measuring intraluminal pressure), while second pressure reading can be from inside the outer shaft (e.g., measuring pressure within the evacuation lumen (e.g., lumen 262)). In other embodiments, more or less pressure readings can be received at 530. At 532, if multiple pressure readings or measurements were taken, a first pressure measurement can be compared to a second pressure measurement. For example, a pressure measurement associated with intraluminal pressure within the body lumen can be compared to a pressure measurement associated with pressure within the evacuation lumen. If the pressures are the same or substantially similar (e.g., the different pressure readings do not differ more than a predetermined amount or percentage from one another) (532: NO), then method 500 can continue to 533, where at least one of the pressure measurements (e.g., intraluminal pressure within body lumen) is evaluated to determine if the pressure reading is within desired pressure parameters (e.g., within a desirable pressure range). If the pressure readings are substantially different from one another (e.g., the different pressure readings differ more than a predetermined amount or percentage from one another, or have a percentage (e.g., 30%) increase or decrease from a nominal operating pressure) (532: YES), or if one or more pressure readings are not within one or more desired pressure parameters (533: NO), information (e.g., an alert) can optionally be presented to the user, at 534, and the supply of ablation medium can be adjusted or terminated, at 535. The information presented to the user can indicate to the user that an error has occurred with the ablation delivery and/or operation of the device. For example, a substantial difference (e.g., difference above a predetermined amount or percentage) between an intraluminal pressure within the body lumen and a pressure within the evacuation lumen (such as the intraluminal pressure being greater than the evacuation lumen pressure) can indicate that a blockage has occurred at some point between the body lumen and the evacuation lumen. With cryogenic delivery systems, such can occur when ice or other solid content blocks a portion of an evacuation lumen. Such blockage can cause a pressure buildup in the body lumen and can result in injury to a patient. Therefore, in such cases, the control unit or other compute device can terminate supply of an ablation medium into the body lumen until the blockage is removed (e.g., via heating coils). In some embodiments, when a pressure measurement is outside of certain pressure parameters (e.g., a predetermined threshold value or range), the control unit can control one or more valves and/or a vacuum source (e.g., vacuum source 130) to evacuate ablation medium from the body lumen so as to reduce pressure buildup within the body lumen, at 536. At 537, if the ablation is completed (537: YES), the method 500 proceeds to terminate the supply of ablation medium, at 539, and then the method 500 can continue to 514. If the ablation is not completed (537: NO), the control unit can continue to monitor pressure measurements and repeat the pressure control protocol.

At 514, the catheter system (e.g., introducer and ablation catheter) can be removed from the body lumen. For a time period (e.g., a few weeks) after the removal of the ablation system, the body's chronic inflammatory response can scar the ablated gallbladder tissue, leading to involution of the lumen and occlusion of the cystic duct. Bile flow can be shut off to the gallbladder, while its blood supply remains uncompromised, resulting in an inert organ.

In some embodiments, as described above, the ablation procedures described herein use a cryogenic ablation medium. In some embodiments, the cryogenic ablation medium is a liquid. In some embodiments, the cryogenic ablation medium is a gas. In some embodiments, the cryogenic ablation medium undergoes a liquid-to-gas phase transition when being delivered using the catheter devices and nozzles disclosed herein. In some embodiments, cryoablation is achieved via the refrigerant property due to the liquid to gas phase change from an ablation medium, such as liquid nitrous oxide, carbon dioxide, and argon. In some embodiments, the phase change of the cryogenic ablation medium is triggered by a sudden reduction in pressure.

FIGS. 11A-11D are illustrations of ablation system implemented as a cryoablation device 1100. The cryoablation device 1100 can be configured to ablate or defunctionalize a gallbladder cavity. The cryoablation device 1100 can include components that are structurally and/or functionally similar to other ablation systems and components thereof described herein (e.g., ablation system 100, catheter system 250, control unit 310, etc.). FIG. 11A shows a side profile view of the cryoablation device 1100, while FIGS. 11B-11D show cross sectional views of various configurations of lumens of inner and outer shafts of the cryoablation device 1100. The cryoablation device 1100 includes a control unit 1110, an outer shaft 1160, an inner shaft 1170, and a pressure sensing lumen 1176. The control unit 1110 includes a processor 1112, a pressure sensor 1115, an input/output interface 1119, and a solenoid valve 1121. In some embodiments, an expandable structure 1179 can hold the inner shaft 1170 in the gallbladder cavity during deployment of an ablation medium. The control unit 1110 is fluidically coupled to an ablation medium supply 1120. In some embodiments, ablation medium can flow from the ablation medium supply to the inner shaft 1170, passing by the solenoid valve 1121.

In some embodiments, the pressure sensing lumen 1176 can terminate at an orifice O that is disposed in the gallbladder cavity, while the pressure sensor 1115 is located outside of the gallbladder cavity. In other words, the pressure sensing lumen 1176 can fluidically couple an interior of the gallbladder cavity to the pressure sensor 1115. The pressure sensor 1115 can be disposed at the control unit 1110 and/or operatively coupled to the control unit 1110. In such a configuration, the pressure sensor 1115 can measure the pressure inside of the gallbladder cavity while being positioned outside of the gallbladder cavity.

The pressure sensing lumen 1176 can be disposed about the inner shaft 1170 or outer shaft 1160 of the catheter system according to one of several different arrangements. For example, in an embodiment, the pressure sensing lumen 1176 can be affixed to the inner shaft 1170, as shown in FIG. 11B. Alternatively, a pressure sensing lumen 1176' can be disposed or defined within the inner shaft 1170, as shown in FIG. 11C. As yet another alternative, a pressure sensing lumen 1176" can be disposed outside the outer shaft 1160, as shown in FIG. 11D. As additional alternatives, a pressure sensing lumen can be integrated into a wall of the inner or outer shaft, coupled to both inner and outer shafts, etc. Pressure can be relieved from the gallbladder cavity via venting paths P that pass through the outer shaft 1160 and to the outside of the cryoablation device 1100. The pressure sensing lumen may also be referred to as a pressure lumen or a sensor lumen.

While not expressly identified in FIGS. 11A-11D, the cryoablation device 1100 includes a lumens defined by inner and outer shafts 1160, 1170 for delivery of an ablation medium and/or evacuation of an ablation medium from the gallbladder cavity. For example, similar to other catheter systems described herein, the inner shaft 1170 can define a lumen (e.g., lumen 272) for delivery of a cryogenic ablation medium to the gallbladder cavity, e.g., via one or more nozzle openings. The outer shaft 1160 can define a lumen (e.g., lumen 262) for evacuating the cryogenic ablation medium from the gallbladder cavity.

Systems, devices, and methods described herein implement a passive evacuation channel and cryogen control system to safely vent cryogen gas from the gallbladder cavity, while ensuring safe operating conditions. During cryogen delivery, a cryogenic ablation medium (e.g., nitrous oxide) expands and evacuates to an external environment (e.g., atmosphere) through an annular space between an inside surface of the outer shaft 1160 and an outer surface of the inner shaft 1170. Resistance in the evacuation channel can cause the gallbladder cavity to distend, to facilitate exposure of tissue within the lumen to the cryogenic ablation medium. The solenoid valve 1121 can be configured to control or regulate delivery of the ablation medium, e.g., from the ablation medium supply 1120, into the gallbladder lumen. For example, the control unit 1110 can control the solenoid valve 1121 to transition from an open state in which ablation medium can be delivered into the gallbladder lumen to a closed state in which ablation medium can be prevented from being delivered into the gallbladder lumen. While a solenoid valve is provided as the example valve herein, it can be appreciated that other types of valves, including mechanically actuated valves, magnetically actuated valves, etc. can be used to control the delivery of the ablation medium into the gallbladder lumen. The control unit 1110, pressure sensor 1115, and solenoid valve 1121 can produce a closed-loop pressure feedback system for maintaining safe operating pressures within the gallbladder cavity. In particular, in response to detecting a pressure within the gallbladder cavity that is greater than a predetermined maximum threshold, the control unit 1110 can control the solenoid valve 1121 to terminate supply of the ablation medium into the gallbladder cavity and/or evacuate via the outer shaft 1160 the ablation medium from the gallbladder cavity to an external environment. Additionally or alternatively, in response to detecting a pressure within the gallbladder cavity that is less than a predetermined minimum threshold, the control unit 1110 can control the ablation medium supply 1120 and/or solenoid valve 1121 to provide additional ablation medium into the gallbladder cavity to sufficiently distend the gallbladder for cryoablation.

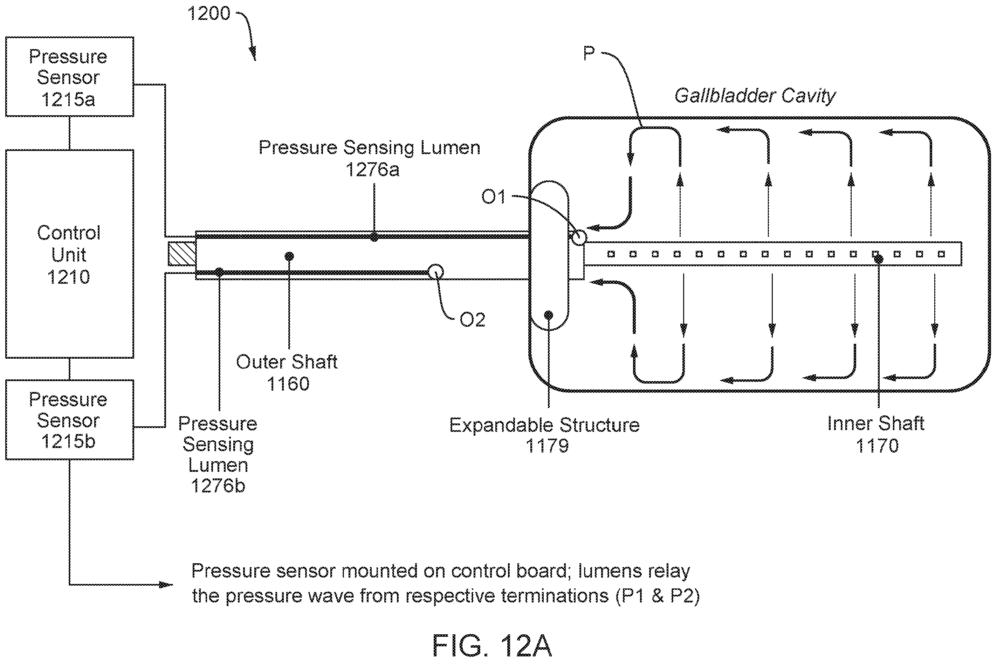

FIGS. 12A-12D are illustrations of an ablation system implemented as a cryoablation device 1200 with multiple pressure sensors, according to an embodiment. The cryoablation device 1200 can be configured to ablate or defunctionalize a gallbladder cavity. The cryoablation device 1200 can include components that are structurally and/or functionally similar to other ablation systems and components thereof described herein (e.g., ablation system 100, catheter system 250, control unit 310, cryoablation device 1100, etc.). For example, the cryoablation device 1200 includes a control unit 1210 with pressure sensors 1215a, 1215b, outer shaft 1260, inner shaft 1270, pressure sensing lumens 1276a and 1276b. In some embodiments, the cryoablation device 1200 can include an expandable structure 1279 that holds the inner shaft 1270 in the gallbladder cavity during deployment of ablation medium. The pressure sensing lumen 1276a includes an orifice O1 deployed in the gallbladder cavity. The pressure sensing lumen 1276a therefore fluidically couples to the pressure sensor 1215a to the gallbladder cavity. The pressure sensing lumen 1262b includes an orifice O2 deployed outside of the gallbladder cavity, e.g., in an evacuation lumen or space. The pressure sensing lumen 1276b therefore fluidically couples the pressure sensor 1215b to the evacuation lumen or other portion of the catheter system. With the placement of orifices O1 and O2, pressure can be measured both inside and outside of the gallbladder cavity.

In some embodiments, redundant pressure sensors can help monitor evacuation pressures or detect a problem during cryoablation therapy. A first pressure sensor (e.g., pressure sensor 1215a) can relay intraluminal pressure from the gallbladder, while a second pressure sensor (e.g., pressure sensor 1215b) can relay the pressure from an evacuation channel or lumen in the outer shaft 1260. Based on the readings or measurements of the pressure sensors 1215a, 1215b, the control unit 1210 can monitor and detect errors in device operation and subsequently halt therapy (e.g., terminate delivery of the ablation medium).

For example, when a blockage has occurred in the evacuation channel, the pressure from pressure sensing lumen 1276a can be greater than the pressure from pressure sensing lumen 1276b. This can indicate that cryoablation medium has caused ice to form at a point along the evacuation channel (e.g., at or near a distal end of the shaft 1260), blocking flow through the evacuation channel. In response to detecting this difference in pressure, the control unit 1210 can be configured to halt therapy (e.g., terminate supply of ablation medium into the gallbladder cavity) such that pressure within the gallbladder cavity does not increase without the capability of venting or evacuating that pressure. In some embodiments, the control unit 1210 can further activate one or more heating coils to melt the ice that has caused the blockage to reopen the evacuation channel and allow for evacuation of ablation medium.

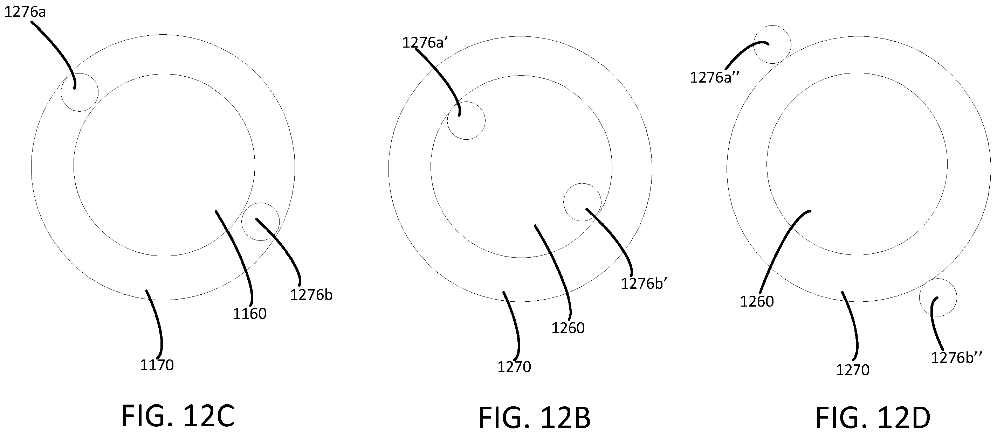

In some embodiments, the pressure sensing lumens 1276a, 1276b can be affixed to the inner shaft 1270, as shown in FIG. 12B. In some embodiments, pressure sensing lumens 1276a', 1276b' can be disposed inside the inner shaft 1270, as shown in FIG. 12C. In some embodiments, the pressure sensing lumens 1276a", 1276b" can be disposed outside the outer shaft 1260, as shown in FIG. 12D. In some embodiments, pressure sensing lumen 1276a can be placed in a different location relative to the inner shaft 1270 than pressure sensing lumen 1276b. In some embodiments, pressure sensing lumens 1276a, 1276b can be placed in still other arrangements, e.g., integrated into a wall of an inner or outer shaft, etc. For example, in some embodiments, pressure sensing lumen 1276a can be integrated into a wall of inner shaft 1270, and pressure sensing lumen 1276b can be integrated into a wall of outer shaft 1260. Alternatively, both pressure sensing lumens 1276a, 1276b can be integrated into and/or defined by a body of inner shaft 1270. Pressure can be relieved from the gallbladder cavity via venting paths P that pass through the outer shaft 1260 and to the outside of the cryoablation device 1200.

While not depicted, the control unit 1210 can include the same components as other control units described herein (e.g., control unit 110, 310, 1110), each having substantially the same structure and/or function. For example, control unit 1210 can include a valve (e.g., a solenoid valve) for controlling or regulating delivery of an ablation medium into the gallbladder cavity.

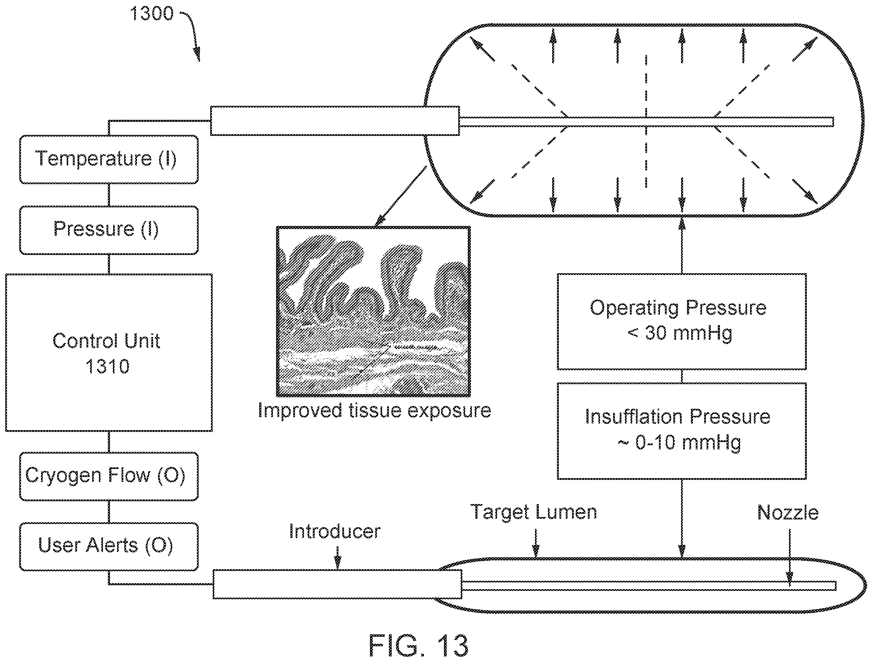

FIG. 13 is an illustration of an ablation system 1300 with a control unit 1310 that facilitates the delivery of a liquid ablation medium (e.g., a cryogenic ablation medium) through a catheter to the gallbladder lumen. The ablation system 1300 can be structurally and/or functionally similar to other ablation systems and components thereof described herein (e.g., ablation system 100, catheter system 250, control unit 310, cryoablation devices 1100, 1200). The control unit 1310 leverages ablation medium flow and evacuation resistance to insufflate the gallbladder lumen to a predetermined pressure or pressure range. At that pressure, the ablation system 1300 can be configured to effectively deliver ablation therapy, e.g., due to the pressure exposing gallbladder tissue for improved ablation contact/efficacy. Distending the gallbladder during ablation is important, as the gallbladder can collapse after bile aspiration, which reduces the ability to deliver therapy to the tissue. Additionally, the epithelial layers of the gallbladder contain mucosal folds, which can shadow tissue during ablation, leading to less efficient cooling or risk for collateral damage. As depicted in FIG. 13, the control unit 1310 can be configured to regulate pressure by monitoring properties of the gallbladder lumen, such as, for example, temperature and pressure, and controlling cryogen flow. In some embodiments, the control unit 1310 can also provide user alerts, such as those described with reference to FIG. 9. In some instances, the insufflation pressure of the gallbladder lumen can be approximately 0-10 mmHg, and the ablation system 1300 can be configured to have an operating pressure that is greater than the insufflation pressure but less than 30 mm Hg. By having an operating pressure that is greater than the insufflation pressure, the ablation device 1300 can distend the gallbladder lumen for an ablation procedure.

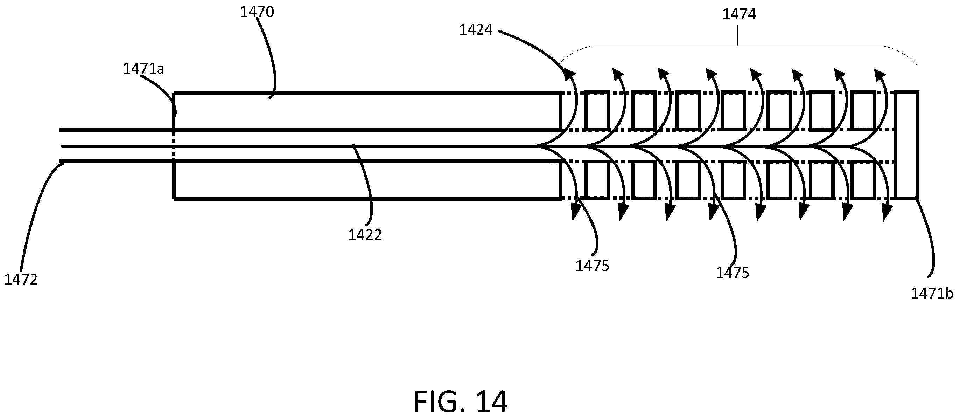

FIG. 14 illustrates an example of an inner shaft 1470 comprising a lumen 1472 and a fenestrated nozzle 1474. In some embodiments, the inner shaft 1470 can include a proximal end 1471a and a distal end 1471b. In some embodiments, the lumen 1472 is sufficiently small keep the cryogenic liquid ablation medium 1422 in a liquid state, with the cryogenic liquid ablation medium 1422 transitioning into a cryogenic gas ablation medium 1424 (i.e., a liquid-to-gas phase transition) as it exits the inner shaft 1470 via a plurality of fenestrations 1475, as shown in FIG. 14 (e.g., due to a pressure drop between inside of lumen 1472 and an inside of the gallbladder). In some embodiments, the cryogenic gas ablation medium 1424 exits the fenestrated nozzle 1474 via the plurality of fenestrations 1475 and ablates the outer surface of the gallbladder lumen once the cryogenic gas ablation medium 1424 upon contact with the tissue.

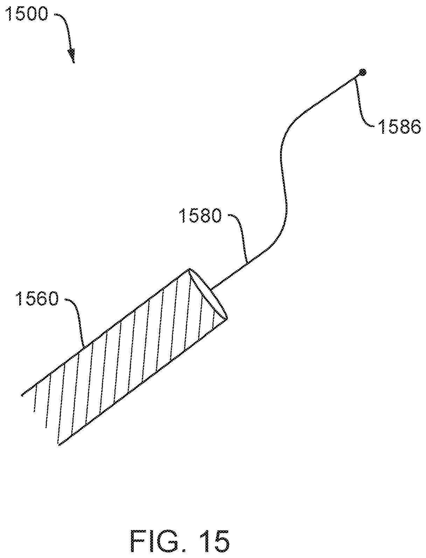

FIGS. 15-16 illustrate examples of temperature measurement probes that can be used to measure surface temperature of tissue and/or temperature within tissue, according to various embodiments of the present disclosure.

Cryoablation destroys tissue through two mechanisms. First, cryoablation produces low temperatures at the target tissue site that induce intracellular ice crystal formation, and subsequent cell death. Second, cryoablation produces reperfusion damage, which is caused by the activation of cell death signaling cascades in response to surrounding cell apoptosis, which helps reinforce cell lethality and is an important part of a cryoablation procedure.

When looking at tissue destruction as a function of temperature, sensors can be employed to measure the temperature at the cryogen source and/or the temperature gradient throughout the tissue. Both temperature measurements are important to evaluating the tissue and status or effectiveness of the ablation. Therefore, temperature measurement systems that provide information on the thermal gradient within the tissue during the procedure are particularly useful in assessing ablation performance.

FIG. 15 illustrates an example of an ablation system 1500. The ablation system 1500 can be structurally and/or functionally similar to other ablation systems and components thereof described herein (e.g., ablation systems 100, 1300, catheter system 250, control unit 310, cryoablation devices 1100, 1200). As depicted in FIG. 15, a probe 1580 (e.g., a temperature measurement probe) can be used with ablation system 1500 for measuring the surface temperature of tissue in a body lumen (e.g., gallbladder lumen). In some embodiments, the probe 1580 can be disposed within a shaft 1560 (e.g., an outer shaft) of a catheter system of the ablation system 1500. In some embodiments, the probe 1580 comprises a temperature sensor 1586 affixed to a compliant "S" shaped spring-loaded arm. In some embodiments, the spring-loaded arm can extend the distal portion of the probe 1580 radially or laterally from a longitudinal axis of the shaft 1560, e.g., to facilitate contact between the temperature sensor 1586 and a tissue surface within the body lumen. In some embodiments, the temperature sensor 1586 can include a thermocouple, thermistor, or infrared heat sensor, and/or any other type of suitable temperature measurement sensor.

In some embodiments, the S-shaped spring-loaded arm of the probe 1580 can be inserted through a delivery lumen (e.g., lumen 262, 264) of the shaft 1560 of the catheter system, in which a maximum radius or lateral extent of the S-shaped spring-loaded arm of the probe 1580 conforms to that of the delivery lumen. When the distal end of the S-shaped spring-loaded arm of the probe 1580 exits the lumen of the shaft 1560, it can transition into an expanded state (e.g., due to spring bias).

In some embodiments, the S-shaped spring-loaded arm 1580 can be made from steel, stainless steel, spring steel, nitinol, a polymer, or a composite with sufficient material elastic and strain bearing properties to enable return to its original confirmation. While the probe 1580 is described as being spring-biased to transition into an expanded structure, it can be appreciated that other mechanisms can be used to bias or extend the distal portion of the probe 1580 toward a tissue surface of the body lumen. For example, a mechanical mechanism (e.g., a pull wire), an electrical mechanism, shape-memory material, etc. can be used to produce a desirable shape and/or expansion of the probe 1580.

The temperature sensor 1586 can be configured to provide temperature data to a processor (e.g., of a control unit, such as control unit 110, 310, etc.) for monitoring and/or analyzing temperature. As described above with reference to FIG. 8, systems, devices, and methods described herein can use such temperature data to track a status of the ablation of the body lumen.

In some embodiments, the temperature sensor 1586 can be configured to penetrate (e.g., pierce) into tissue of the body lumen. For example, the probe 1580 can terminate in a needle or other suitable structure, and the temperature sensor can be mounted to and/or integrated into the needle. The temperature sensor 1586, by being disposed within the tissue, can be configured to obtain temperature measurements of the tissue that are less impacted by the extreme temperature of the ablation medium disposed within the body lumen.

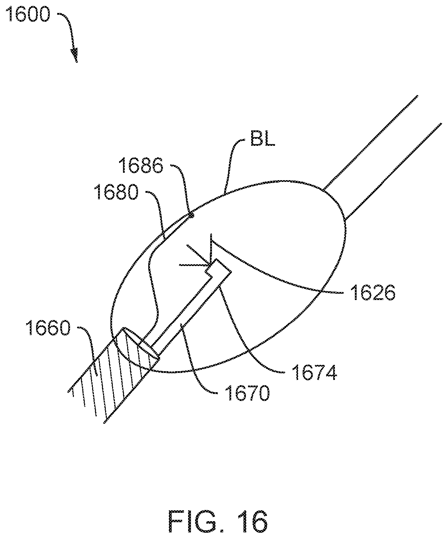

FIG. 16 illustrates an example of an ablation system 1600. The ablation system 1600 can be structurally and/or functionally similar to other ablation systems and components thereof described herein (e.g., ablation systems 100, 1300, 1500, catheter system 250, control unit 310, cryoablation devices 1100, 1200). The ablation system 1600 can include a probe 1680 (e.g., a temperature measurement probe), according to some embodiments. As shown in FIG. 16, the ablation device 1600 includes an outer shaft 1660 and an inner shaft 1670. The probe 1680 and include a spring-loaded arm and a temperature sensor 1686. The inner shaft 1670 includes a cryogen spray nozzle 1674 configured to uniformly disperse cryogen 1626 onto a targeted area disposed within and surrounded by the outer shaft 1660. According to various embodiments, the probe 1680 can measure the temperature of a targeted area as the cryogen spray nozzle 1674 disperses the cryogen 1626 during the ablation procedure. The distal end of the probe 1680 including the temperature sensor 1686 can be disposed within a body lumen BL to deliver cryoablation therapy. The probe 1680 can be functionally and/or structurally similar to probes 280, 1580, as described above.

Similar to ablation system 1500, the temperature sensor 1686 can be configured to provide temperature data to a processor (e.g., of a control unit, such as control unit 110, 310, etc.) for monitoring and/or analyzing temperature. As described above with reference to FIG. 8, systems, devices, and methods described herein can use such temperature data to track a status of the ablation of the body lumen.

FIGS. 17-19 show illustrations of inner shafts of an ablation catheter system with cryogen supply valves for providing cryogen to a target area, according to various embodiments. In particular, FIGS. 17-19 illustrate examples of cryogen supply lumen valve mechanisms located on the distal end of a cryogen catheter. In some embodiments, the distal valve mechanism is comprised of a cryogen supply lumen and an actuate ball to impede cryogen flow distal to the mechanism. FIG. 17 is an illustration of an inner shaft 1770 of a catheter system, according to an embodiment. The inner shaft 1770 can be structurally and/or functionally similar to other inner shafts as described herein (e.g., inner shaft 270, etc.). The inner shaft 1770 includes a lumen 1772, and a valve cable 1778a attached to a valve ball 1778b. The ball 1778b is located on the distal side of the valve interface, which mechanically seals the lumen 1772 when seated against a conforming mating face 1777. In some embodiments, the valve geometry can be a sphere, a cube, cone, cylinder, triangular prism, torus, helix, ovoid, or any other 3D body with sufficient structure impede cryogen flow.

FIG. 18 is an illustration of an inner shaft 1870 of a catheter system, according to an embodiment. The inner shaft 1870 can be structurally and/or functionally similar to other inner shafts as described herein (e.g., inner shaft 270, 1770, etc.). The inner shaft 1870 includes a lumen 1872, a valve cable 1878a attached to a valve ball 1878b, and a return spring 1896. FIG. 18 also depicts cryogen 1826 exiting the inner shaft 1870. In some embodiments, the valve geometry can be held in a normally closed state with either the return spring 1896 or a cable tension system. In either case, a mechanical opposing force is used to create a proper seal between the valve ball 1878b and a conforming mating face 1877, thereby impeding fluid flow and sealing the lumen 1872. In some embodiments, the valve geometry can be actuated, either manually or robotically, with a drive wire/rod, pneumatic/hydraulic pressure, electromagnetic force, and or motor to open and close the valve, with or without the need for a return mechanism.

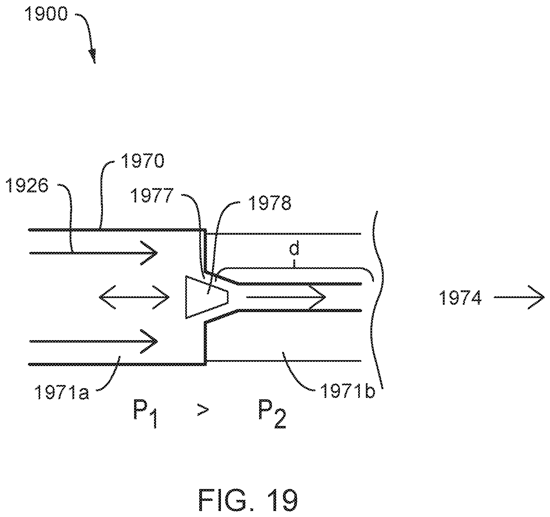

In some embodiments, the valve geometry can be located on the proximal side of the conforming mating face and leverage the pressure of the cryogen supply line (e.g., pressure within ablation medium delivery lumen, e.g., lumen 272 of an inner shaft 270) to stay in a normally closed state. In some embodiments, a driving force can be applied to break the seal between the valve geometry and the conforming mating face, thereby allowing the flow of cryogen. For example, FIG. 19 is an illustration of an inner shaft 1970 of a catheter system, according to an embodiment. The inner shaft 1970 can be structurally and/or functionally similar to other inner shafts as described herein (e.g., inner shaft 270, 1770, 1870, etc.). The inner shaft 1970 includes an upstream section 1971a upstream of a valve 1978 and a downstream section 1971b downstream of the valve 1978, a conforming mating face 1977, and a nozzle 1974 (location of the nozzle 1974 is indicated by arrow). FIG. 19 also depicts cryogen 1926 in the inner shaft and downstream of the valve 1978.

In some embodiments, the cryogen supply line volume 1971b (e.g., volume of the ablation medium delivery lumen, e.g., lumen 272 of an inner shaft 270) distal to the valve 1978 is sufficiently small to minimize residual volume pressure buildup, such as that described above with reference to valve 278 depicted in FIG. 3. In some embodiments, the distance d of the cryogen supply line distal to the valve is about 0-10 centimeters (cm) or any inclusive range. In some embodiments, the valve 1978 can consist of a valve body and a rotating valve geometry that opens/closes an orifice that facilitates the flow of cryogen 1926.

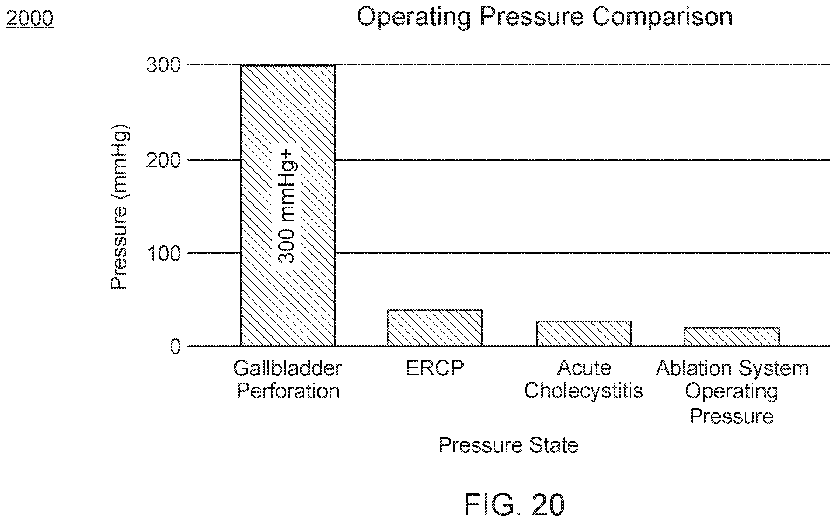

FIG. 20 is a chart 2000 depicting operational pressure data for a cryoablation device in accordance with embodiments described herein in comparison to the thresholds for gallbladder perforation, endoscopic retrograde cholangiopancreatography (ERCP), and acute cholecystitis. As can be seen from the plot on FIG. 20, the cryoablation device according to embodiments described herein operates at pressures lower than those that would cause the aforementioned conditions.

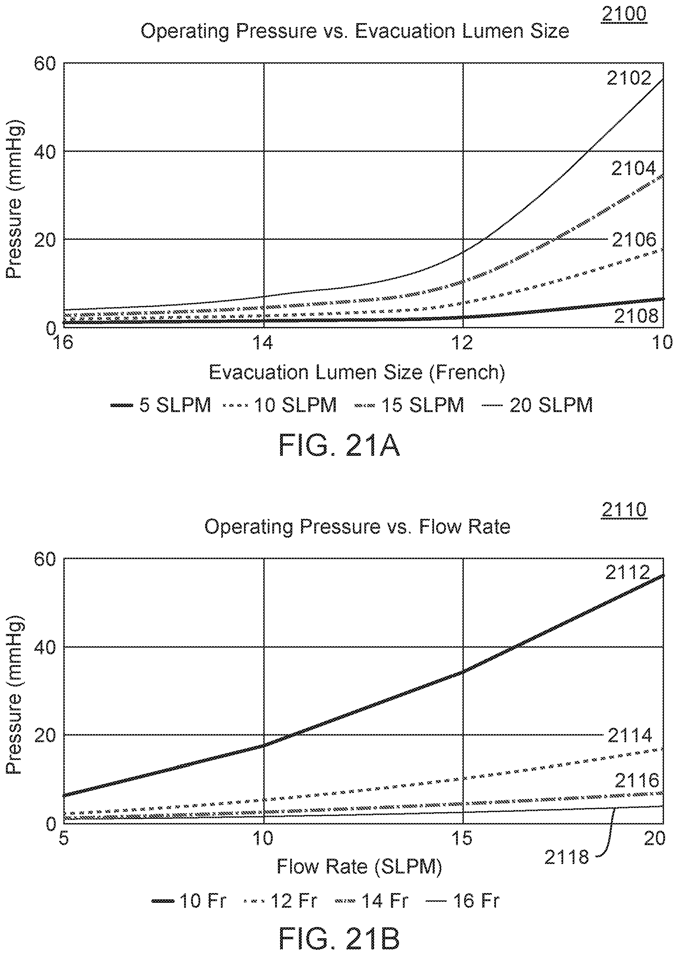

FIGS. 21A-21B are graphs that show backpressure within an ablation device (e.g., such as any of the ablation devices described herein) as a function of evacuation lumen size and flow rate of the ablation medium delivery (e.g., cryogen flow rate). Graph 2100 depicts pressure as a function of evacuation lumen size, given different flow rates, with line 2102 corresponding to a flow rate of 20 standard liters per minute (SLPM), line 2104 corresponding to a flow rate of 15 SLPM, line 2106 corresponding to a flow rate of 10 SLPM, and line 2108 corresponding to a flow rate of 5 SLPM. Graph 2110 depicts pressure as a function of flow rate, given different evacuation lumen sizes, with line 2112 corresponding to an evacuation lumen size of 10 French (Fr), line 2114 corresponding to an evacuation lumen size of 12 Fr, line 2116 corresponding to an evacuation lumen size of 14 Fr, and line 2118 corresponding to an evacuation lumen size of 16 Fr. These French sizes for the evacuation lumen are based on circular cross-sectional areas; therefore, an evacuation lumen size of 10 Fr corresponds to a cross-sectional area of approximately 8.7 mm², an evacuation lumen size of 12 Fr corresponds to a cross-sectional area of approximately 12.6 mm², and so on and so forth. With lumen sizes ranging from 10 to 16 Fr, operating pressures can range from near atmospheric to about 55 mm Hg gauge when evacuating between 5 and 20 SLPM. Based on this data, a minimum evacuation lumen size is about 12 Fr and a maximum flow rate is about 20 SLPM for achieving backpressure of less than about 20 mmHg, assuming a 6 Fr ablation catheter (e.g., inner shaft 270) with a nozzle. The 6 Fr ablation catheter can have a 2 mm outer diameter and a cross-sectional area of approximately 3.1 mm². The outer diameter of the ablation catheter and the inner diameter of the introducer (e.g., outer shaft 260) determine a size of an annular evacuation space. Accordingly, with a 6 Fr ablation catheter and a 12 Fr evacuation lumen size (or inner diameter of the introducer), the annular evacuation cross-sectional area is approximately 9.4 mm². As such, in some embodiments, systems, devices, and methods described herein provide an annular evacuation space that has an cross-sectional area of at least 9 mm².

One of the primary difficulties with consideration to device safety is controlling the intraluminal pressure buildup associated with the phase-change of the liquid nitrous oxide ablation medium sprayed within the gallbladder or other targeted ablation area. More specifically, if a pressure build-up event is detected, there should be a safe way to ensure that the pressure does not continue to build up and is properly evacuated. In the situation where a pressure build up event is occurring, a large concern is the remaining liquid nitrous oxide located within the delivery lumen due to the fact that is has the potential to continue to deliver a large volume of gas, depending on the total volume of the "uncontrolled supply line" or the segment of the supply line that is beyond any mechanical valve, which can cut the flow of the supply line. Accordingly, it can be beneficial to minimize the concern of supply line pressure by monitoring the pressure and adjusting the device accordingly.

According to various embodiments of the present disclosure, a cryoablation device can be configured to control a flow of cryogen within the cryogen delivery lumen a cryoablation device. For example, the cryoablation device can include a valve mechanism or other type of device that can be used to open and/or close an area in the cryogen delivery lumen (e.g., lumen 272 of an inner shaft or ablation catheter 270) thereby restricting or allowing a flow of cryogen past a distal end of the valve mechanism and into a targeted ablation area (e.g., gallbladder). According to various embodiments, the cryoablation device can be in data communication with a computing device that is configured to monitor pressure within a gallbladder lumen or other targeted lumen. As will be discussed below, pressure can be released by controlling cryogen flow via a control valve and/or other component. According to various embodiments, the cryoablation device can comprise one or more pressure sensors in data communication with the computing device. The pressure sensors are configured to measure the pressure within the gallbladder lumen or other targeted lumen.

It should be emphasized that the above-described embodiments of the present disclosure are merely possible examples of implementations set forth for a clear understanding of the principles of the disclosure. Many variations and modifications may be made to the above-described embodiment(s) without departing substantially from the principles of the disclosure. All such modifications and variations are intended to be included herein within the scope of this disclosure and protected by the following claims.

Also, various concepts may be embodied as one or more methods, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

As used herein, the terms "about" and/or "approximately" when used in conjunction with numerical values and/or ranges generally refer to those numerical values and/or ranges near to a recited numerical value and/or range. In some instances, the terms "about" and "approximately" may mean within ± 10% of the recited value. For example, in some instances, "about 100 [units]" may mean within ± 10% of 100 (e.g., from 90 to 110). The terms "about" and "approximately" may be used interchangeably.

Some embodiments described herein relate to a computer storage product with a non-transitory computer-readable medium (also may be referred to as a non-transitory processor-readable medium) having instructions or computer code thereon for performing various computer-implemented operations. The computer-readable medium (or processor-readable medium) is non-transitory in the sense that it does not include transitory propagating signals per se (e.g., a propagating electromagnetic wave carrying information on a transmission medium such as space or a cable). The media and computer code (also may be referred to as code or algorithm) may be those designed and constructed for the specific purpose or purposes. Examples of non-transitory computer-readable media include, but are not limited to, magnetic storage media such as hard disks, floppy disks, and magnetic tape; optical storage media such as Compact Disc/Digital Video Discs (CD/DVDs), Compact Disc-Read Only Memories (CD-ROMs), and holographic devices; magneto-optical storage media such as optical disks; carrier wave signal processing modules; and hardware devices that are specially configured to store and execute program code, such as Application-Specific Integrated Circuits (ASICs), Programmable Logic Devices (PLDs), Read-Only Memory (ROM) and Random-Access Memory (RAM) devices. Other embodiments described herein relate to a computer program product, which may include, for example, the instructions and/or computer code disclosed herein.

The systems, devices, and/or methods described herein may be performed by software (executed on hardware), hardware, or a combination thereof. Hardware modules may include, for example, a general-purpose processor (or microprocessor or microcontroller), a field programmable gate array (FPGA), and/or an application specific integrated circuit (ASIC). Software modules (executed on hardware) may be expressed in a variety of software languages (e.g., computer code), including C, C++, Java^{®}, Ruby, Visual Basic^{®}, and/or other object-oriented, procedural, or other programming language and development tools. Examples of computer code include, but are not limited to, microcode or micro-instructions, machine instructions, such as produced by a compiler, code used to produce a web service, and files containing higher-level instructions that are executed by a computer using an interpreter. Additional examples of computer code include, but are not limited to, control signals, encrypted code, and compressed code.

## Claims

1. An apparatus, comprising:
a shaft including a nozzle disposable within a body lumen of a subject, the shaft defining:
an ablation medium delivery lumen in fluid communication with the nozzle, the ablation medium delivery lumen configured to deliver an ablation medium to the nozzle, the nozzle configured to distribute the ablation medium throughout the body lumen such that the ablation medium contacts and ablates tissue within the body lumen;
a first pressure lumen having a distal opening that terminates in the body lumen when the nozzle is disposed in the body lumen, the first pressure lumen configured to couple a first pressure sensor disposed proximal to the shaft to the body lumen such that the first pressure sensor can measure an intraluminal pressure of the body lumen; and
a second pressure lumen having a distal opening that terminates in an evacuation lumen configured to evacuate a portion of the ablation medium from the body lumen, the second pressure lumen configured to couple a second pressure sensor disposed proximal to the shaft to the evacuation lumen such that the second pressure sensor can measure a pressure in the evacuation lumen.

2. The apparatus of claim 1, wherein the shaft is an inner shaft, the apparatus further comprising:
an outer shaft defining the evacuation lumen,
the inner shaft disposable within the evacuation lumen.

3. The apparatus of claim 2, wherein the outer shaft is configured to be placed through an opening into the body lumen, the apparatus further comprising:
an expandable structure disposed about a distal portion of the outer shaft, the expandable structure configured to transition into an expanded state within the body lumen, the expandable structure in the expanded state having an outer diameter greater than a diameter of the opening into the body lumen to prevent proximal movement of the outer shaft.

4. The apparatus of claim 1, further comprising:
a probe including a distal end including a temperature sensor, the distal end of the probe disposable within the body lumen such that the temperature sensor can be placed in contact with tissue lining the body lumen to measure a temperature of the tissue.

5. The apparatus of claim 4, wherein the shaft is an inner shaft, the probe being insertable into the body lumen through a lumen defined by an outer shaft, the distal end of the probe configured to deflect away from a longitudinal axis of the shaft and toward the tissue lining the body lumen when the distal end of the probe is extended distal to the outer shaft.

6. The apparatus of claim 1, wherein the nozzle includes a plurality of fenestrations that are in fluid communication with the ablation medium delivery lumen, the plurality of fenestrations distributed about a circumference of the nozzle and along an axial length of the nozzle such that the nozzle is configured to distribute the ablation medium throughout the body lumen.

7. The apparatus of claim 1, wherein the ablation medium is a cryogenic ablation medium, the nozzle configured to induce the cryogenic ablation medium to change from a liquid to a gas.

8. The apparatus of claim 1, wherein the body lumen is a gallbladder lumen, the apparatus further comprising a cystic duct occluder disposed at a distal end of the shaft and configured to occlude a cystic duct of the subject.

9. The apparatus of claim 1, wherein the evacuation lumen is configured to evacuate an ablation medium from the body lumen in response to an intraluminal pressure of the body lumen being above a predetermined threshold.

10. The apparatus of claim 1, wherein the ablation medium delivery lumen and the evacuation lumen are collectively configured to maintain an intraluminal pressure of the body lumen within a predetermined range.

11. The apparatus of claim 11, wherein the predetermined range is less than about 50 mm Hg.

12. The apparatus of claim 1, further comprising a valve disposed within the ablation medium delivery lumen, the valve configured to transition between an open state and a closed state, the valve in the open state configured to allow the ablation medium to be delivered to the nozzle and in the closed state configured to prevent the ablation medium from being delivered to the nozzle.

## Patentansprüche

1. Vorrichtung, umfassend:
einen Schaft, der eine Düse aufweist, die in einem Körperlumen eines Subjekts angeordnet werden kann, wobei der Schaft definiert:
ein Ablationsmediumzufuhrlumen in Fluidverbindung mit der Düse, wobei das Ablationsmediumzufuhrlumen dazu ausgelegt ist, der Düse ein Ablationsmedium zuzuführen, wobei die Düse dazu ausgelegt ist, das Ablationsmedium derart im Körperlumen zu verteilen, dass das Ablationsmedium das Gewebe in dem Körperlumen kontaktiert und ablatiert;
ein erstes Drucklumen mit einer distalen Öffnung, die in das Körperlumen mündet, wenn die Düse in dem Körperlumen angeordnet ist, wobei das erste Drucklumen dazu ausgelegt ist, einen ersten Drucksensor, der proximal zu dem Schaft angeordnet ist, derart mit dem Körperlumen zu koppeln, dass der erste Drucksensor einen intraluminalen Druck des Körperlumens messen kann; und
ein zweites Drucklumen mit einer distalen Öffnung, die in ein Evakuierlumen mündet, das dazu ausgelegt ist, einen Abschnitt des Ablationsmediums aus dem Körperlumen zu evakuieren, wobei das zweite Drucklumen dazu ausgelegt ist, einen zweiten Drucksensor, der proximal zu dem Schaft angeordnet ist, derart mit dem Evakuierlumen zu koppeln, dass der zweite Drucksensor einen Druck in dem Evakuierlumen messen kann.

2. Vorrichtung nach Anspruch 1, wobei der Schaft ein Innenschaft ist, wobei die Vorrichtung ferner umfasst:
einen Außenschaft, der das Evakuierlumen definiert,
den Innenschaft, der in dem Evakuierlumen angeordnet werden kann.

3. Vorrichtung nach Anspruch 2, wobei der Außenschaft dazu ausgelegt ist, durch eine Öffnung in das Körperlumen platziert zu werden, wobei die Vorrichtung ferner umfasst:
eine expandierbare Struktur, die um einen distalen Abschnitt des Außenschafts angeordnet ist, wobei die expandierbare Struktur dazu ausgelegt ist, in dem Körperlumen in einen expandierten Zustand überzugehen, wobei die expandierbare Struktur in dem expandierten Zustand einen Außendurchmesser hat, der größer ist als ein Durchmesser der Öffnung in das Körperlumen, um eine proximale Bewegung des Außenschafts zu verhindern.

4. Vorrichtung nach Anspruch 1, ferner umfassend:
eine Sonde, die ein distales Ende mit einem Temperatursensor aufweist, wobei das distale Ende der Sonde derart in dem Körperlumen angeordnet werden kann, dass der Temperatursensor in Kontakt mit dem Gewebe platziert werden kann, welches das Körperlumen auskleidet, um eine Temperatur des Gewebes zu messen.

5. Vorrichtung nach Anspruch 4, wobei der Schaft ein Innenschaft ist, wobei die Sonde durch ein von einem Außenschaft definierten Lumen in das Körperlumen einsetzbar ist, wobei das distale Ende der Sonde dazu ausgelegt ist, von einer Längsachse des Schafts weg und zum Gewebe, das das Körperlumen auskleidet, hin abgelenkt zu werden, wenn sich das distale Ende der Sonde distal zu dem Außenschaft erstreckt.

6. Vorrichtung nach Anspruch 1, wobei die Düse eine Vielzahl von Fenestrationen aufweist, die mit dem Ablationsmediumzufuhrlumen in Fluidverbindung sind, wobei die Vielzahl von Fenestrationen um einen Umfang der Düse und entlang einer axialen Länge der Düse derart verteilt sind, dass die Düse dazu ausgelegt ist, das Ablationsmedium überall im Körperlumen zu verteilen.

7. Vorrichtung nach Anspruch 1, wobei das Ablationsmedium ein kryogenes Ablationsmedium ist, wobei die Düse dazu ausgelegt ist, den Übergang des kryogenen Ablationsmediums von einer Flüssigkeit in ein Gas zu induzieren.

8. Vorrichtung nach Anspruch 1, wobei das Körperlumen ein Gallenblasenlumen ist, wobei die Vorrichtung ferner einen Gallengangverschluss umfasst, der an einem distalen Ende des Schafts angeordnet und dazu ausgelegt ist, einen Gallengang des Subjekts zu verschließen.

9. Vorrichtung nach Anspruch 1, wobei das Evakuierlumen dazu ausgelegt ist, ein Ablationsmedium aus dem Körperlumen in Reaktion darauf zu evakuieren, dass ein intraluminaler Druck des Körperlumens über einem vorbestimmten Schwellenwert liegt.

10. Vorrichtung nach Anspruch 1, wobei das Ablationsmediumzufuhrlumen und das Evakuierlumen gemeinsam dazu ausgelegt sind, einen intraluminalen Druck des Körperlumens in einem vorbestimmten Bereich zu halten.

11. Vorrichtung nach Anspruch 11, wobei der vorbestimmte Bereich kleiner ist als etwa 50 mm Hg.

12. Vorrichtung nach Anspruch 1, ferner umfassend ein Ventil, das in dem Ablationsmediumzufuhrlumen angeordnet ist, wobei das Ventil dazu ausgelegt ist, zwischen einem offenen Zustand und einem geschlossenen Zustand zu wechseln, wobei das Ventil in dem offenen Zustand dazu ausgelegt ist, zu erlauben, dass das Ablationsmedium der Düse zugeführt wird, und in dem geschlossenen Zustand dazu ausgelegt ist, zu verhindern, dass das Ablationsmedium der Düse zugeführt wird.

## Revendications

1. Appareil, comprenant :
un arbre comprenant une buse pouvant être disposée dans une lumière corporelle d'un sujet, l'arbre définissant :
une lumière d'alimentation en milieu d'ablation en communication fluidique avec la buse, la lumière d'alimentation en milieu d'ablation étant configurée pour fournir un milieu d'ablation à la buse, la buse étant configurée pour distribuer le milieu d'ablation dans toute la lumière corporelle de telle sorte que le milieu d'ablation entre en contact avec et ablate le tissu dans la lumière corporelle ;
une première lumière de pression ayant une ouverture distale qui se termine dans la lumière corporelle lorsque la buse est disposée dans la lumière corporelle, la première lumière de pression étant configurée pour coupler un premier capteur de pression disposé à proximité de l'arbre à la lumière corporelle de telle sorte que le premier capteur de pression puisse mesurer une pression intraluminale de la lumière corporelle ; et
une deuxième lumière de pression ayant une ouverture distale qui se termine dans une lumière d'évacuation configurée pour évacuer une partie du milieu d'ablation de la lumière corporelle, la deuxième lumière de pression étant configurée pour coupler un deuxième capteur de pression disposé à proximité de l'arbre à la lumière d'évacuation de telle sorte que le deuxième capteur de pression puisse mesurer une pression dans la lumière d'évacuation.

2. Appareil selon la revendication 1, l'arbre étant un arbre interne, l'appareil comprenant en outre :
un arbre externe définissant la lumière d'évacuation,
l'arbre interne pouvant être disposé à l'intérieur de la lumière d'évacuation.

3. Appareil selon la revendication 2, l'arbre externe étant configuré pour être placé à travers une ouverture dans la lumière corporelle, l'appareil comprenant en outre :
une structure extensible disposée autour d'une partie distale de l'arbre externe, la structure extensible étant configurée pour passer à un état étendu à l'intérieur de la lumière corporelle, la structure extensible dans l'état étendu ayant un diamètre extérieur supérieur à un diamètre de l'ouverture dans la lumière corporelle pour empêcher un mouvement proximal de l'arbre externe.

4. Appareil selon la revendication 1, comprenant en outre :
une sonde comprenant une extrémité distale comprenant un capteur de température, l'extrémité distale de la sonde pouvant être disposée dans la lumière corporelle de telle sorte que le capteur de température puisse être placé en contact avec le tissu recouvrant la lumière corporelle afin de mesurer une température du tissu.

5. Appareil selon la revendication 4, l'arbre étant un arbre interne, la sonde pouvant être insérée dans la lumière corporelle à travers une lumière définie par un arbre externe, l'extrémité distale de la sonde étant configurée pour se dévier à l'écart d'un axe longitudinal de l'arbre et vers le tissu recouvrant la lumière corporelle lorsque l'extrémité distale de la sonde est étendue distalement par rapport à l'arbre externe.

6. Appareil selon la revendication 1, la buse comprenant une pluralité de fenestrations qui sont en communication fluidique avec la lumière d'alimentation en milieu d'ablation, la pluralité de fenestrations étant réparties autour d'une circonférence de la buse et le long d'une longueur axiale de la buse de telle sorte que la buse est configurée pour distribuer le milieu d'ablation dans toute la lumière corporelle.

7. Appareil selon la revendication 1, le milieu d'ablation étant un milieu d'ablation cryogénique, la buse étant configurée pour amener le milieu d'ablation cryogénique à passer d'un état liquide à un état gazeux.

8. Appareil selon la revendication 1, la lumière corporelle étant une lumière de la vésicule biliaire, l'appareil comprenant en outre un dispositif d'occlusion de canal cystique disposé à une extrémité distale de l'arbre et configuré pour occlure un canal cystique du sujet.

9. Appareil selon la revendication 1, la lumière d'évacuation étant configurée pour évacuer un milieu d'ablation de la lumière corporelle en réponse à une pression intraluminale de la lumière corporelle supérieure à un seuil prédéterminé.

10. Appareil selon la revendication 1, la lumière d'alimentation en milieu d'ablation et la lumière d'évacuation étant configurées collectivement pour maintenir une pression intraluminale de la lumière corporelle dans une plage prédéterminée.

11. Appareil selon la revendication 11, la plage prédéterminée étant inférieure à environ 50 mm Hg.

12. Appareil selon la revendication 1, comprenant en outre une valve disposée à l'intérieur de la lumière d'alimentation en milieu d'ablation, la valve étant configurée pour passer d'un état ouvert à un état fermé, la valve dans l'état ouvert étant configurée pour permettre au milieu d'ablation d'être fourni à la buse et dans l'état fermé étant configurée pour empêcher le milieu d'ablation d'être fourni à la buse.
